# EUROPEAN PATENT SPECIFICATION

(11) **EP 2 934 564 B1**
(45) Date of publication and mention of the grant of the patent: **23.05.2018**
(21) Application number: 13821470.5
(22) Date of filing: 20.12.2013
(51) Int. Cl.: A61K 38/17, A61K 35/54, A61P 35/00, A61P 25/00

(54) **ANTISECRETORY FACTOR (AF) FOR USE IN THE TREATMENT OF GLIOBLASTOMA**
ANTISEKRETORISCHEM FAKTOR (AF) ZUR VERWENDUNG IN DER BEHANDLUNG VON GLIOBLASTOM
UN FACTEUR ANTISÉCRÉTOIRE (FA) POUR L'UTILISATION DANS LE TRAITEMENT DU GLIOBLASTOME

(30) Priority: 20.12.2012 SE 1251473
(43) Date of publication of application: 28.10.2015
(73) Proprietor: Lantmännen AS-Faktor AB, 10425 Stockholm (SE)
(72) Inventor: HANSSON, Hans-Arne, S-436 58 Hovås (SE)
(74) Representative: Valea AB
(86) International application number: PCT/EP2013/077747
(87) International publication number: WO 2014/096384

(56) References cited:
- US-A1- 2003 143 285
- US-A1- 2007 009 575
- US-A1- 2012 093 716

## Description

### Field of invention

The present invention relates to the use of an antisecretory factor (AF) protein, peptide, derivative, homologue, and/or fragment thereof, having equivalent functional activity, and/or a pharmaceutically active salt thereof, as an adjuvant for the treatment of glioblastoma and/or for optimizing delivery and cellular uptake of a pharmaceutical substance and/or formulation, such as an anticancer drug, immune therapy, radiation therapy or a gene delivery to a glioblastoma tumor cell.

In another aspect, the present disclosure relates to the design of a new and reliable diagnostic and/or prognostic tool for monitoring and/or verifying and/or enhancing the therapeutic control of a glioblastoma in a subject suffering from glioblastoma.

### Background of the invention

Glioblastoma multiforme (GBM) is the most common and most aggressive malignant primary brain tumor in humans, involving glial cells and accounting for a large fraction of all intracranial tumors. It is the most prevalent neoplastic form of the about 120 different types of primary brain tumors. The GBM annual incidence is 2-3 cases per 100,000 people in Europe and North America. According to the WHO classification of the tumors of the central nervous system, the standard name for this brain tumor is "glioblastoma", and it presents in several variants.

Glioblastoma multiforme (GBM) is recognized as the most common and lethal form of central nervous system cancer. Currently used surgical techniques, chemotherapeutic agents, immune therapy and radiotherapy strategies have done little in extending the life expectancies of patients diagnosed with GBM. The difficulty in treating this malignant disease lies both in its inherent complexity and in its elaborated mechanisms of drug resistance.

Glioblastoma has the worst prognosis of any central nervous system (CNS) malignancy, despite multimodality treatment consisting of surgical resection of as much of the tumor as possible, with concomitant or sequential chemotherapy, radiotherapy, antiangiogenic therapy, immune therapy, gamma knife radiosurgery, and symptomatic management with corticosteroids. Prognosis is very poor, with a median survival time of approximately one year and the disease is almost invariably fatal, as only about 3 % survive for more than 3 years.

Glioblastoma multiforme tumors, also named Grade IV, are characterized by the presence of areas of necrotizing tissue that are surrounded by anaplastic cells with numerous mitosis and endothelial proliferation. This characteristic, as well as the presence of hyperplastic abnormal blood vessels, differentiates the tumor from Grade III astrocytomas, which do not have these features.

There are several subtypes of glioblastoma. Ninety-seven percent of tumors in the 'classical' subtype carry extra copies of the Epidermal Growth Factor Receptor (EGFR) gene, and most have higher than normal expression of EGFR, whereas the gene TP53, which is often mutated in glioblastoma, is rarely mutated in this subtype. In contrast, the proneural subtype often has high rates of alterations in TP53, and in PDGFRα, the gene encoding α-type of platelet-derived growth factor receptor, and in IDHI, the gene encoding isocitrate dehydrogenase-1. The mesenchymal subtype is characterized by high rates of mutations or other alterations in NF1, the gene encoding Neurofibromatosis type 1, fewer alterations in the EGFR gene and less expression of EGFR than other types.

GBM tumors usually appear in the cerebral white matter, grow quickly, and can become very large before causing symptoms. Less than 10% form more slowly, following dedifferentiation of low-grade astrocytoma or anaplastic astrocytoma. These are called secondary GBM tumors and are more common in younger patients (mean age 45 versus 62 years).The tumor may extend into the meninges or ventricular wall, leading to high protein content in the cerebrospinal fluid (CSF) (> 100 mg/dL), as well as an occasional pleocytosis of 10 to 100 cells, mostly lymphocytes. Malignant cells carried in the CSF may spread (rarely) to the spinal cord or cause meningeal gliomatosis. However, metastasis of GBM beyond the central nervous system is extremely unusual. About 50% of GBM tumors occupy more than one lobe of a hemisphere or are bilateral. Tumors of this type usually arise from the cerebrum and may rarely exhibit the classic infiltration across the corpus callosum, producing a butterfly (bilateral) glioma.

The tumor may take on a variety of appearances, depending on the amount of hemorrhage, necrosis, and/or its age. A CT scan will usually show an inhomogeneous mass with a hypodense center and a variable ring of enhancement surrounded by edema. Mass effect from the tumor and edema may compress the ventricles and cause e.g. hydrocephalus as well as mechanical distortion and herniation.

The formations of abnormal, dysfunctional, tumor vasculature and glioblastoma stem-like cells (GSCs) are believed to be the major components of the inability to treat GBM tumors effectively. Furthermore, the tumor cells are cocooned in a protective glycocalyx coating, preventing the access of e.g. drugs to reach and exert actions on the tumor cells.

Treatment of primary brain tumors and brain metastases consists of curative, symptomatic and palliative therapies.

The primary supportive agents for symptomatic therapy are anticonvulsants and corticosteroids.

Curative and palliative treatment includes surgery, radiation therapy, immune therapy and chemotherapy. A maximally feasible resection is usually performed along with radiation and chemotherapy. Gross total resection of tumor is associated with a better prognosis.

Unfortunately, GBM tumors are known to contain zones of tissue exhibiting hypoxia which are highly resistant to radiotherapy. Tumor stem cells have been demonstrated to prevail and are resistant to available therapy. Various approaches to chemotherapy, immunotherapy and radiosensitizers have been pursued with limited success to date.

Further, among other treatments, also gene transfer and protein therapeutic treatments are tested at present.

The median survival time from the time of diagnosis without any treatment is 3 months and with full therapy, as utilized today, about a year. Only every fifth survives for 2 years after modern, extensive therapy. Increasing age (> 60 years of age) carries a worse prognostic risk. Death is usually due to cerebral edema and/or increased intracranial pressure and, additionally, due to mass effects that impair blood circulation and cause brain herniation.

Thus there is a long felt need for an improved and/or optimized treatment for GBM tumors.

Antisecretory factor (AF) is a 41 kDa protein that originally was described to provide protection against diarrhea diseases and intestinal inflammation (for a review, see Lange and Lönnroth, 2001). The antisecretory factor (AF) protein has been sequenced and its cDNA cloned. The antisecretory activity seems to be mainly exerted by a peptide located between the amino acid positions 35 and 50 on the antisecretory factor (AF) protein sequence and comprising at least 4-16, such as 4, 6, 7, 8 or 16 amino acids of the consensus sequence. Immunochemical and immunohistochemical investigations have revealed that the antisecretory factor (AF) protein is present and may also be synthesized by most tissues and organs in a body. Synthetic peptides, comprising the antidiarrhoeic sequence, have prior been characterized (WO 97/08202; WO 05/030246). Antisecretory factor (AF) proteins and peptides have previously been disclosed to normalize pathological fluid transport and/or inflammatory reactions, such as in the intestine and the choroid plexus in the central nervous system after challenge with the cholera toxin (WO 97/08202). Food and feed with the capacity to either induce endogenous synthesis of AF or uptake of added AF have therefore been suggested to be useful for the treatment of edema, diarrhea, dehydration and inflammation in WO 97/08202. WO 98/21978 discloses the use of products having enzymatic activity for the production of a food that induces the formation of antisecretory factor (AF) proteins. WO 00/038535 further discloses food products enriched in native antisecretory factor (AF) proteins as such.

Antisecretory factor (AF) proteins and fragments thereof have also been shown to improve the repair of nervous tissue, and proliferation, apoptosis, differentiation, and/or migration of stem and progenitor cells and cells derived thereof in the treatment of conditions associated with loss and/or gain of cells (WO 05/030246) and to be equally effective in the treatment and/or prevention of intraocular hypertension (WO 07/126364), as for the treatment and/or prevention of compartment syndrome (WO 07/126363).

What is more, the present inventors recently showed that AF is able to monitor and/or beneficially affect the structure, distribution and multiple functions of lipid rafts, receptors and/or caveolae in membranes and could thus be employed for the treatment and/or prevention of structural disorganization and dysfunction of lipid rafts and/or caveolae in cell membranes (WO 07/126365).

The present inventors have further been able to prove that the same antisecretory factor (AF) protein, peptides and fragments thereof can intervene in the biological activation of transmembrane proteins, e.g. NKCC1 through FAK and CAP, and that they can thus directly regulate the pathological activity of the ion channel in pathological and/or perturbed cells, effectively normalizing the intracellular pressure and transmembrane protein function in said cell, and thus allowing an improved uptake of drugs used in e.g. cancer therapy (WO 2010/093324).

The present application for the first time discloses that antisecretory factor (AF) proteins, peptides, homologues and/or fragments thereof can further be used both in symptomatic, curative and palliative therapies for glioblastoma. In particular, they can be used as an adjuvant and/or to optimize drug and gene delivery, as well as chemotherapy, immunotherapy and radiotherapy in the treatment of glioblastoma.

### Summary of the present invention

The present invention is defined by the claims. The present application for the first time discloses a long sought for effective approach for treating GBM tumors. It surprisingly discloses that antisecretory factor AF can be used both in symptomatic, curative and palliative therapies for glioblastoma. In particular, it can be used as an adjuvant and/or to optimize drug and gene delivery, as well as chemotherapy, immunotherapy and radiotherapy in the treatment of glioblastoma.

As can be seen in the experimental section, the present application for the first time discloses a fragment of an antisecretory factor (AF) protein as shown in SEQ ID NO: 1 (AF), having equivalent activity and comprising an amino acid sequence as shown in SEQ ID NO: 2 (AF-6), for use in lowering the elevated intracranial pressure caused by a glioblastoma tumor to an acceptable level, about 20 mm Hg or lower, as well as for promoting and/or improving entrance of a chemotherapeutic substance into glioblastoma tumor cells.

The present invention therefore relates to an antisecretory factor (AF) protein as shown in SEQ ID NO: 1 (AF), and/or a homologue, and/or fragment thereof having equivalent activity and at least comprising an amino acid sequence as shown in SEQ ID NO: 2 (AF-6), and/or a pharmaceutically active salt thereof, for use in the treatment of glioblastoma (a GBM tumor). In one embodiment, it relates to a food and/or food supplement, enriched in said antisecretory factor (AF) protein and/or a homologue and/or fragment thereof, for use in the treatment of glioblastoma (a GBM tumor).

The antisecretory factor (AF) protein and/or a homologue and/or fragment thereof and/or a pharmaceutically active salt thereof, and/or a food and/or food supplement, enriched in said antisecretory factor (AF) protein and/or homologue and/or fragment thereof, as defined herein, can be used for manufacturing a composition for use in symptomatic, curative and/or palliative therapy of glioblastoma and/or in a method for symptomatic, curative and/or palliative treatment of glioblastoma.

In particular, the antisecretory factor (AF) protein and/or a homologue and/or fragment thereof and/or a pharmaceutically active salt thereof, and/or a food and/or food supplement, enriched in said antisecretory factor (AF) protein and/or homologue and/or fragment thereof can be used in optimizing delivery and/or cellular uptake of a further pharmaceutical substance and/or formulation and/or gene delivery for treating glioblastoma (a GBM tumor). On the other hand, the antisecretory factor (AF) protein and/or a homologue and/or fragment thereof and/or a pharmaceutically active salt thereof, and/or a food and/or food supplement, enriched in said antisecretory factor (AF) protein and/or homologue and/or fragment thereof can be used as a drug in the treatment of glioblastoma (a GBM tumor) in itself.

### Figure legends

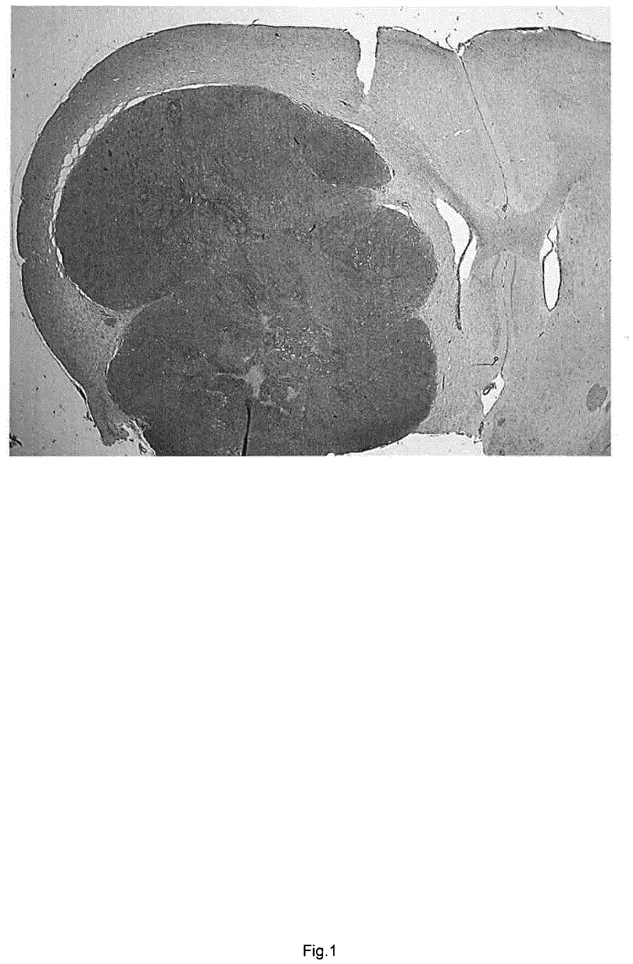

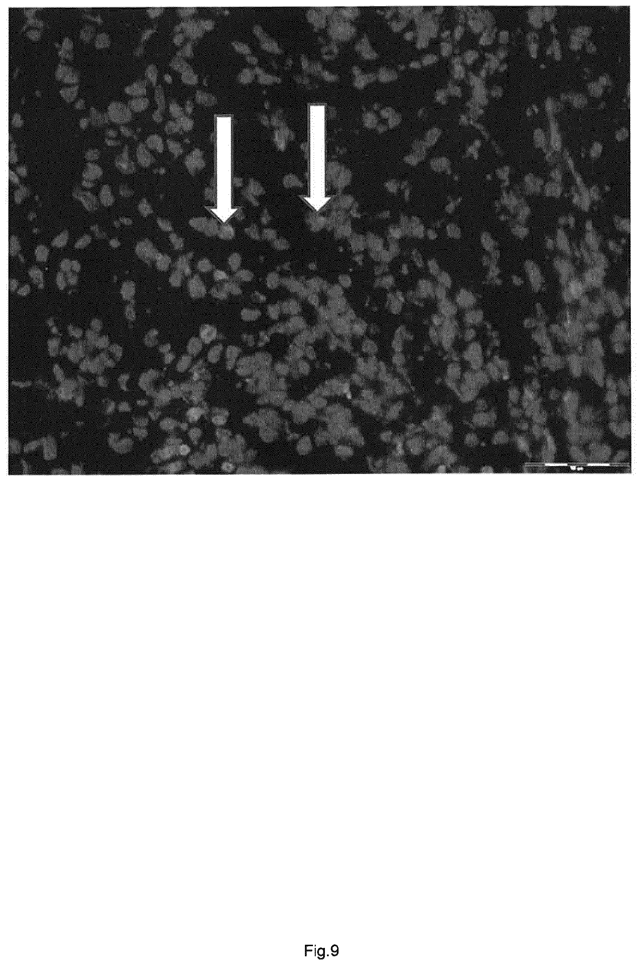

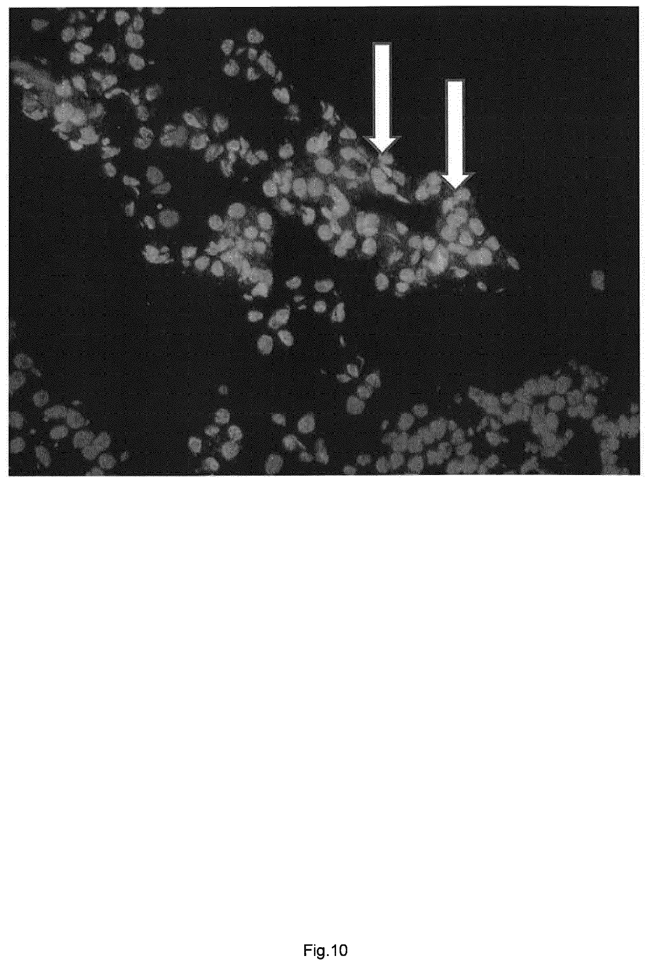

**Figure 1****:**
   Cross section of a rat brain with a RG2-N32 glioblastoma (dark staining; day 22) in the center, delimited by edematous brain tissue (light orange). Note that the right hemisphere in the center of the picture is enlarge, causing a midline shift as well as dislocation and deformation of the midline and the lateral ventricles.
**Figure 2****:**
   Higher magnification of Fig.1. The highly cellular tumor is invading the brain tissue by finger-like extensions, rendering the tumor difficult to delimit.
**Figure 3****:**
   Fluorescence micrograph of a RG2-N32 glioblastoma, processed for demonstration of the glycocalyx, which delimits each tumor cell. Each tumor cell is enclosed by a glycocalyx rich in α-N-acetylgalactosamine (green/marked by white arrows) as well as to a lesser extent by α-N-acetylglucosamine (red). Note the red staining of the erythrocytes. Two large blood vessels are marked by black arrows.
**Figure 4****:**
   An anaesthetized rat is resting on its abdomen. The skull is exposed and the light guides to two Samba pressure sensors enter the brain through holes in the cranium. The two light guides (arrows) are fixed by holding instruments when performing experiments, but in this photo kept free for ease of illustration.
**Figure 5****:**
   The elevated intracranial pressure is visualized to be in the order of 30 mm Hg during the half an hour recording in a rat with a glioblastoma (day 22). The corresponding pressure in a normal rat is 5.3 ± 2.1 mm Hg. Both the expanding tumor mass and the brain edema contribute to the raised pressure.
**Figure 6****:**
   The intracranial pressure in a rat with a glioblastoma will increase with time and eventually cause extensive brain deformation and dislocation as well as herniation and circulatory collapse. There is presently hardly any options available enabling rescue of the animal from the deleterious effects by the tumor expansion and the brain edema. The animal was killed at the time indicated by an arrow.
**Figure 7****:**
   The intracranial pressure in a rat with a glioblastoma is decreasing, starting about 12 minutes after nasal administration of AF-16 (4 mg/kg bw). The red and blue lines show that the pressure drop is going on concomitantly in either brain hemisphere, attaining in half an hour a pressure in the order of 20 mm Hg, which is considered to be an acceptable level.
**Figure 8****:**
   The intracranial pressure in a rat with a glioblastoma is reduced, starting about 15 min after nasal instillation of AF-6 (1 mg/kg bw). After almost half an hour 17.7 mm Hg is attained.
**Figures 9** **and** **10****:**
   Rats with a glioblastoma in their right brain hemisphere had either the vehicle, water (Fig. 9), or AF-16 (4 mg/kg bw) instilled nasally I h prior to an intravenous injection of the tracer doxorubicin (10 mg/kg bw). The brains were 30 min later removed from the anaesthetized animals and processed for fluorescence microscopy. Pretreatment with AF-16 (Fig. 10) increased both the intensity of the staining and the frequency of stained tumor cell nuclei (exemplified cells pointed at with white arrows), as compared to that achieved after pretreatment with the vehicle (Fig. 9). This result documents that AF-16 facilitates the uptake and binding of the marker to intracellular tumor cell structures, i.e. the target of the treatment.

### Definitions and abbreviations

### Abbreviations

IFP: interstitial fluid pressure;
PBS: phosphate buffered saline;
AF: antisecretory factor,
Full-length AF protein (as shown in SEQ ID NO: 1)
AF-6: a hexa peptide CHSKTR (as shown in SEQ ID NO: 2);
AF-16: a peptide composed of the amino acids VCHSKTRSNPENNVGL (as shown in SEQ ID NO: 3);
AF-8: a septa peptide VCHSKTR (as shown in SEQ ID NO: 4);
Octa peptide IVCHSKTR (as shown in SEQ ID NO: 5);
Penta peptide HSKTR (as shown in SEQ ID NO: 6).
SPC: Specially Processed Cereals
RTT: Method for measuring a standardized secretion response in rat small intestine, as published in SE 9000028-2 (publication number 466331) for measuring content of AF (ASP).

### Definitions

Proteins are biological macromolecules constituted by amino acid residues linked together by peptide bonds. Proteins, as linear polymers of amino acids, are also called polypeptides. Typically, proteins have 50-800 amino acid residues and hence have molecular weights in the range of from about 6,000 to about several hundred thousand Dalton or more. Small proteins are called peptides, polypeptides, or oligopeptides. The terms "protein", "polypeptide", "oligopeptide" and "peptide" may be used interchangeably in the present context. Peptides can have very few amino acid residues, such as between 2-50 amino acid residues (aa).

A "pharmaceutical composition", in the present context, refers to a composition comprising a therapeutically active amount of an antisecretory factor (AF) protein, optionally in combination with a pharmaceutically active excipient, such as a carrier or a vehicle. Said pharmaceutical composition is formulated for the appropriate route of administration, which may vary depending on the condition of the patient, as well as on other factors, such as age or preferred choice. A pharmaceutical composition comprising an antisecretory factor (AF) protein can serve as a drug delivery system. The pharmaceutical composition upon administration presents the active substance to the body of a human or an animal. Said pharmaceutical composition may be in the form of e.g. tablets, pills, lozenges, capsules, stool pills, gels, solutions, etc., but is not limited thereto.

The term "pharmaceutically active salt", refers to a salt of an antisecretory factor (AF) protein, peptide, or polypeptide, or a homologue and/or fragment thereof which may be any salt derived therefrom, based on so-called Hofmeiser's series. Other examples of pharmaceutically active salts comprise triflouroacetate, acetate and lysine chloride, the invention is not limited thereto.

The term "antisecretory" refers in the present context to inhibiting or decreasing secretion and/or fluid transfer. Hence, the term "antisecretory factor (AF) protein" refers to a class of proteins capable of inhibiting or decreasing or otherwise modulating fluid transfer as well as secretion in a body.

In the present context, "equivalent activity" relates to the biological effect of the antisecretory factor (AF) protein, peptide, or polypeptide, or a homologue, derivative and/or fragment thereof, i.e. its capacity for improving therapy and/or use in treating glioblastoma. Standardized examples for testing and/or measuring such a capacity are well known in the field of the art. Examples are given in the experimental section of this application, such as in examples 1 - 4.

In the present context, the terms an "Antisecretory factor protein", "antisecretory factor (AF) protein", "AF- protein", AF, or a homologue, derivative or fragment thereof, may be used interchangeably with the term "antisecretory factors" or "antisecretory factor proteins" as defined in WO 97/08202, and refer to an antisecretory factor (AF) protein or a peptide or a homologue, derivative and/or fragment thereof having antisecretory and/or equivalent functional and/or analogue activity, or to a modification thereof not altering the function of the polypeptide. Hence, it is to be understood that an "antisecretory factor", "antisecretory factor protein", "antisecretory peptide", "antisecretory fragment", or an "antisecretory factor (AF) protein" in the present context, also can refer to a derivative, homologue or fragment thereof. These terms may all be used interchangeably in the context of the present invention. Furthermore, in the present context, the term "antisecretory factor" may be abbreviated "AF". Antisecretory factor (AF) protein in the present context also refers to a protein with antisecretory properties as previously defined in WO97/08202 and WO 00/38535. Antisecretory factors have also been disclosed e.g. in WO 05/030246. Also intended by the term antisecretory factor are native antisecretory factors in egg yolk enriched in antisecretory factors as disclosed in SE 900028-2 and WO 00/38535, as further described below.

A "medical food", in the present context, refers to a food, a food supplement, or a food for special dietary use, which has been prepared with an antisecretory factor (AF) protein, or alternatively, has the capability to induce synthesis and/or activation of endogenous AF. Said food may be any suitable food, in fluid or solid form, such as a liquid or a powder, or any other suitable foodstuff. Examples of such matter may be found in WO 0038535 or WO 91/09536.

A "nebulizer", in the present context, refers to a medical device that delivers liquid medication in the form of a mist to the airways.

The term "aerosol" in the present context refers to a gaseous suspension of fine solid or liquid particles.

In the present context, the term "cytostaticum" is used, as well as "cytostatic drugs", "cytostatica", "cytostatic agents" or "cytostatic compounds", the terms are interchangeable and relate to drugs which are used in cancer therapy and are typically administered to patients undergoing chemotherapy (cytostatic agents are so called anticancer drugs). Cytostatic agents are substances which check the growth of pathological cells, and also of normal cells. Such substances are therefore used for the chemotherapeutical treatment of tumors, but also for post-operational and/or post-radiation treatment after removal of a tumor. Cytostatic agents can come in liquid, powder or granular form, optionally also deep-frozen. The person skilled in the art will adjust the choice and dosage of cytostatic agent from a plethora of cytostatic agents commercially available.

In the present context, the term "cytotoxic" is used, as well as "cytotoxic drugs", "cytotoxica", "cytotoxic agents" or "cytotoxic compounds", the terms are interchangeable and relate to drugs which are used in cancer therapy and are typically administered to patients undergoing chemotherapy (cytotoxic agents are so called anticancer drugs). Cytotoxic agents are substances which are toxic to cells and thereby may check the growth of pathological cells, and also of normal cells. Such substances are therefore used for the chemotherapeutical treatment of tumors, but also for post-operational treatment after removal of a tumor or post-radiation therapy. Cytotoxic agents can come in liquid, powder or granular form, optionally also deep-frozen. The person skilled in the art will adjust the choice and dosage of cytotoxica from a plethora of cytotoxica commercially available.

In the present context, the term "adjuvant" is used to describe a pharmacological and/or immunological agent that modifies the effect of other agents. Adjuvants of in the present application enhance or optimize the recipient's use of the active substance administered, thus minimizing the necessary amount of said substance.

Glioblastoma multiforme tumors can in the present context either be giant cell glioblastoma or gliosarcoma.

### Detailed description of the invention

The present invention relates to the use of an antisecretory factor (AF) protein, peptide, derivative, homologue, and/or fragment thereof, having equivalent functional activity, and/or a pharmaceutically active salt thereof, for treating glioblastoma (a GBM tumor) and/or for optimizing delivery and/or cellular uptake of a pharmaceutical substance and/or formulation, such as an anticancer drug, radiation therapy, therapy relying on immunological mechanisms or a gene delivery to a glioblastoma tumor cell.

The present application for the first time discloses a long sought for effective means and/or approach for treating GBM tumors. It surprisingly discloses that antisecretory factors can be used both in symptomatic, curative and palliative therapies for glioblastoma. In particular, they can be used as an adjuvant and/or to optimize drug and gene delivery, as well as chemotherapy, immunotherapy and radiotherapy in the treatment of glioblastoma.

In particular, the antisecretory factor (AF) protein and/or a homologue and/or fragment thereof and/or a pharmaceutically active salt thereof, and/or a food and/or food supplement, enriched in said AF protein and/or homologue and/or fragment thereof is herein used for optimizing blood circulation and/or oxygen tension in a GBM tumor, facilitating effects of radiation therapy as well as immune therapy and/or chemotherapy.

The present invention therefore relates to an antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, for use in the treatment of glioblastoma (a GBM tumor). In one embodiment, it relates to a food and/or food supplement, enriched in said antisecretory factor (AF) protein and/or a homologue and/or fragment thereof, for use in the treatment of glioblastoma (a GBM tumor).

In another aspect, the present invention relates to the design of a new and reliable diagnostic and/or prognostic tool for monitoring and/or verifying and/or enhancing the therapeutic control of a glioblastoma (a GBM tumor) in a subject suffering from glioblastoma. An antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, is herein used for optimizing delivery and/or cellular uptake of a pharmaceutical substance and/or formulation to a GBM tumor cell. Said pharmaceutical substance and/or formulation is in the present context selected from the group consisting of anticancer drug, antitumor drug, radiation therapy, immunological substances and/or cells and antibiotic substance, a drug targeting posttraumatic injury, a drug targeting neurodegeneration, and a drug against inflammatory conditions.

Examples well known in the field are selected from but not limited to the group comprising an alkylating agent, such as temozolomide and Gliadel®, which is an implantable alkylating carmustine wafer. In addition, incorporated are antibodies to vascular endothelial growth factor.

The best long term results for the therapy of glioblastoma have so far been achieved by using the combination of temozolomide and radiation. It ought to be stressed that the efficacy of radiation is improved by high tissue tension of oxygen, which facilitates the generation of free radicals. Thus, the present invention relates to an antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, for use in optimizing delivery and/or cellular uptake of temozolomide, alternatively in combination with radiation to a GBM tumor cell.

The present invention equally relates to the manufacture of a pharmaceutical formulation comprising an antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, and temozolomide, for use in treating glioblastoma, optionally in combination with radiation.

An obstacle to both chemotherapy and to radiation is that the individual GBM tumor cells are enclosed by a coat, most evidently composed of the glycocalyx lining cells extracellular surface. Thereby, the efficacy of the therapy is hampered, often to such an extent that there are limited short and long term effects. Glioblastoma tumors have a very rich and elaborated network of blood vessels, but the enclosing of the tumor cells strongly reduces the delivery into the tumor cells of chemotherapeutic agents as well as of e.g. oxygen.

The present invention relates to an antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, for use in overcoming the obstacles for chemotherapeutics agents and oxygen to reach their targets, the individual GBM tumor cells.

The present invention equally relates to the manufacture of a pharmaceutical formulation comprising antisecretory factor (AF) protein, peptide, derivative, homologue, and/or fragment thereof, having equivalent functional activity, or a modification thereof not altering the function of the polypeptide, and/or a pharmaceutically active salt thereof, for use in overcoming the obstacles for chemotherapeutics agents and oxygen to reach their targets, the individual GBM tumor cells.

Brain edema is an early event in the growth of glioblastoma and is to a, with time increasing, extent commonly not limited to the tumor region but evolving throughout the brain. This means that the content within the rigid cranium is expanding not only by the tumor growth and occurrence of hemorrhages but also due to edema formation. As the rigid cranium resists expansion the intracranial pressure is increasing, restricting the blood circulation as well as deforming and distorting the brain tissue. Raised intracranial pressure will cause the patient neurological and psychiatric symptoms of increasing severity. An important goal in the treatment of glioblastoma is thus to reduce the brain edema and thereby the intracranial pressure, facilitating e.g. the blood circulation, tentatively improving the access of chemotherapeutic agents and e.g. oxygen and consequently free radicals to the tumor cells. A further beneficial effect is to improve the well-being of patients suffering from glioblastoma by reducing the brain edema and the intracranial pressure.

Antisecretory factor (AF) effectively regulates and/or normalizes abnormal activity of specific ion and/or fluid channels in perturbed and pathological cells, thereby effectively normalizing the dimensions and the intracellular pressure in the cell, which in turn also can lead to normalizing the interstitial pressure in a perturbed tissue, and thus potentially allowing an improved cellular uptake of a pharmaceutical substance, such as drugs used in the treatment of glioblastoma.

As shown in the experimental section, AF-16, a fragment of an antisecretory factor (AF) protein as shown in SEQ ID NO: 1 (AF), having equivalent activity and comprising an amino acid sequence as shown in SEQ ID NO: 2 (AF-6), could be demonstrated to lower the raised intracranial pressure prevailing in the head of animals with experimentally induced glioblastoma. This highly beneficial effect was reproducibly induced by the administration of AF-16. It ought to be stressed that AF-16 reduced the intracranial pressure not only in the hemisphere with the tumor but also in the contralateral hemisphere, which is edematous in spite of not containing any tumor. Furthermore, a critical effect by the administration of AF-16 h efficiently improved the uptake by the tumor cell nuclei of the marker substance doxorubicin. These two beneficial effects could as well be demonstrated to be achieved with the substance AF-6, also a fragment of an antisecretory factor (AF) protein as shown in SEQ ID NO: 1 (AF), having equivalent activity and comprising an amino acid sequence as shown in SEQ ID NO: 2 (AF-6). Additional experiments indicated that AF-16 both reduced the high intracranial pressure in mice with experimentally induced glioblastoma and also facilitated the uptake and binding of doxorubicin to tumor cell nuclei.

The present invention consequently for the first time discloses the use of an AF protein as shown in SEQ ID NO: 1 (AF), and/or a homologue and/or fragment thereof having equivalent activity and comprising an amino acid sequence as shown in SEQ ID NO: 2 (AF-6), and/or a pharmaceutically active salt thereof, for the manufacture of a pharmaceutical composition and/or a medical food for use in the treatment of glioblastoma (a GBM tumor), either for use in symptomatic, curative and/or palliative treatment of glioblastoma.

The present invention relates to the use of a pharmaceutical composition comprising an antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, for the manufacture of a pharmaceutical composition for optimizing delivery and/or cellular uptake of a second or further pharmaceutical substance and/or formulation for use in treating glioblastoma. Typically, said second or further pharmaceutical substance and/or formulation comprises an anticancer drug, radiation therapy, antimicrobial substance, antibiotic substance, and/or a drug targeting posttraumatic injury, neurodegeneration, and/or an inflammatory condition.

In yet another aspect, the present invention relates to a pharmaceutical composition comprising an antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, in combination with a second or further pharmaceutical substance and/or formulation for treating glioblastoma, wherein said second or further pharmaceutical substance and/or formulation is selected from the group consisting of an anticancer drug, radiation therapy, antibiotic substance, and a drug targeting posttraumatic injury, neurodegeneration, or an inflammatory condition, as such, and to its use in medicine, in particular to its use in the treatment of glioblastoma.

Furthermore, said pharmaceutical composition can of course comprise two or more antisecretory factor (AF) proteins, fragments, derivates, or combinations thereof, as well as further comprising a pharmaceutically acceptable excipient.

In particular, the AF protein and/or a homologue and/or fragment thereof and/or a pharmaceutically active salt thereof, and/or a food and/or food supplement, enriched in said AF protein and/or homologue and/or fragment thereof are used in optimizing delivery and/or cellular uptake of a further pharmaceutical substance in the form of nano-particles and/or formulations thereof in the treatment of glioblastoma (a GBM tumor).

For example, said second or further pharmaceutical substance and/or formulation can be selected from the group consisting of an anticancer drug, a cytostaticum, genetic material, radiation therapy, antimicrobial substance, antibiotic substance, antiviral substance, immunologically active compound and a drug targeting posttraumatic injury, neurodegeneration, or an inflammatory condition.

Further, said pharmaceutical composition can comprise two or more antisecretory factor (AF) proteins and/or homologues and/or fragments thereof and/or pharmaceutically active salts thereof, as well as a pharmaceutically acceptable excipient.

In a presently preferred embodiment, an antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, is shown to be able to overcome cellular barriers in malignant and/or pathological cells in glioblastoma, such as a protective glycocalix coating, and can thus be used as an adjuvant for lowering a required drug dosage, alternatively for maximizing the dosage effect of said pharmaceutical substance and/or formulation. In consequence, said above described antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, and/or a pharmaceutical composition comprising it, can be used to minimize toxic or unwanted side effects of said pharmaceutical substance and/or formulation in the treatment of glioblastoma.

Furthermore, a food and/or food supplement, enriched in antisecretory factor (AF) protein and/or homologue and/or fragment thereof, as described herein, is preferably provided as egg yolk enriched in naturally occurring antisecretory factors.

Thus, the present invention also relates to a pharmaceutical composition comprising a food and/or food supplement, enriched in an antisecretory factor (AF)-protein and/or homologue and/or fragment thereof as described herein, such as egg yolk enriched in naturally occurring antisecretory factors, in combination with an anti-cancer drug, such as temozolomide, as well as its general use in medicine and in particular, its use in the treatment of glioblastoma (a GBM tumor).

In another, equally preferred embodiment, an antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, can be produced endogenously by the patient after intake of a food and/or a food for special dietary use that induces the uptake, formation and/or release of an antisecretory factor (AF) protein.

The present invention relates to the use of a pharmaceutical composition comprising an antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), and/or a pharmaceutically active salt thereof, for the manufacture of a pharmaceutical composition for optimizing delivery and/or cellular uptake of a second or further pharmaceutical substance and/or formulation for treating glioblastoma. AF and the second or further pharmaceutical substance and/or formulation can be administered together or in alternating succession. They can be co-formulated or administered in separate formulations.

A presently preferred embodiment of the present invention is further the use of a pharmaceutical composition comprising an antisecretory factor (AF) protein, a homologue, derivative, peptide and/or fragment thereof, according to the present invention, for the manufacture of a pharmaceutical composition for optimizing radiation therapy in the treatment of glioblastoma.

What is more, based on the transient and reversible nature of the lowering of the cellular IFP, the clinician can easily envision an administration routine wherein an antisecretory factor (AF) protein, a homologue, derivative, peptide and/or fragment thereof, according to the present invention, is administered in optimally timed intervals that are so adjusted that the IFP in the target cells are lowered just in time for the administration of the second or further pharmaceutical substance and/or formulation during the treatment regimen for a glioblastoma patient.

Thus, another, equally preferred embodiment relates to a method or to an administration dosage regimen for optimized delivery and/or cellular uptake of a second or further pharmaceutical substance and/or formulation, wherein said second or further pharmaceutical substance and/or formulation comprises an anti-cancer drug, radiation therapy, antibiotic substance, and/or a drug targeting posttraumatic injury, neurodegeneration, or an inflammatory condition suitable for treating glioblastoma.

The antisecretory factor (AF) protein, a homologue, derivative, peptide and/or fragment thereof, according to the present invention, and the second or further pharmaceutical substance and/or formulation can be administered together or in alternating succession. They can be co-formulated or administered in separate formulations.

A pharmaceutical composition according to the present invention can be formulated for intraocular, intranasal, oral, local, subcutaneous and/or systemic administration, and can be intended for administration as a spray, aerosol, and/or by an inhaler or nebulizer. The pharmaceutical composition according to the present invention can in one context be administrated by application topically, locally in situ, orally, in the nose, subcutaneously and / or systemically via blood vessels or via the respiratory tract.

When the pharmaceutical composition and/or medical food is formulated for administration systemically to the blood, dosage regimen is selected at a dose of e.g. AF-16 (SEQ ID NO:3) equivalent to 0.1 µg to 10 mg per application and kg body weight and day, preferably 1-1000 µg per application and kg body weight and day, and said administration is performed either as a single dose or as multiple daily applications.

Consequently, the present invention also relates to a pharmaceutical composition comprising an antisecretory factor (AF) protein as shown in SEQ ID NO: 1 (AF), and/or a homologue and/or fragment thereof having equivalent activity and comprising an amino acid sequences as shown in SEQ ID NO: 2 (AF-6), such as SEQ ID NO: 1 (AF), SEQ ID NO: 2 (AF-6), SEQ ID NO:3 (AF-16), and/or a pharmaceutically active salt thereof in combination with an anti-cancer drug, such as temozolomide, as well as its general use in medicine and in particular, its use in the treatment of glioblastoma (a GBM tumor).

In another aspect, the present invention relates to the use of a pharmaceutical composition comprising an antisecretory factor (AF) protein, a homologue, derivative, peptide and/or fragment thereof, according to the present invention, or a pharmaceutically active salt thereof, for the manufacture of a pharmaceutical composition for optimizing delivery and/or cellular uptake of a second or further pharmaceutical substance and/or formulation for the treatment and/or prevention of glioblastoma.

Again, various administration doses and routes are envisioned suitable for the intended purpose of treatment as well as the patient's age, gender, condition etc.

The very wide range of effective dose regimes utilized indicates that the risks for side effects and unexpected complications are minimal. Thus, the present invention will enable the treatment of excessive loads on cells and tissues as wells as to treat a patient with a wide range of doses suiting the individual response and the severity of the illness and/or the discomfort.

The present invention further in one presently preferred instance relates to a method for improved drug design characterized by testing the response of cells or tissues, subject to treatment of glioblastoma to a substance or a pharmaceutical formulation referred to in the present application and estimating the influence of an antisecretory factor (AF) protein, a homologue, derivative, peptide and/or fragment thereof, according to the present invention, or combinations thereof on the cellular uptake of said substance or formulation by e.g. measuring the amount of phosphorylated FAK.

Any of the above described methods can typically alternatively be conducted in a cellular system or in a test organism. The methods are also equally applicable in *in vivo, in situ,* and *in silico systems.*

### Standard methods :

- for improved drug design,
- for screening for and/or evaluating potential AF inhibitory and/or enhancing substances,
- for evaluating efficacy and/or verifying functional activity of new or known antisecretory factor (AF) proteins, peptides, derivatives, homologues, and/or fragments thereof, having equivalent functional activity, and/or a pharmaceutically active salt thereof,
   are well known in the art.

### The antisecretory factor

The antisecretory factor is a class of proteins that occurs naturally in the body. The human antisecretory factor AF protein is a 41 kDa protein, comprising 382-288 amino acids when isolated from the pituitary gland. The active site with regard to the beneficial effect on treatment of glioblastoma according to the present invention can be localized to the protein in a region close to the N-terminal of the protein, in particular localized to amino acids 1-163 of SEQ ID NO 1, more specifically to amino acid positions 35 - 50 on the antisecretory factor (AF) protein sequence. The biological effect of AF is exerted by any peptide or polypeptide comprising at least 6 amino acids, SEQ ID NO: 2 (AF-6), of said consensus sequence, or a modification thereof not altering the function of the polypeptide and/or peptide.

The present inventors have shown that the antisecretory factor is to some extent homologous with the protein S5a, and Rpn10, which constitutes a subunit of a constituent prevailing in all cells, the 26 S proteasome, more specifically in the 19 S/PA 700 cap. In the present invention, antisecretory factor (AF) proteins are defined as a class of homologue proteins having the same functional properties. Antisecretory factor is also highly similar to angiocidin, another protein isoform known to bind to thrombospondin-1 and associated with cancer progression.

Homologues, derivatives and fragments of antisecretory factor (AF) proteins and/or peptides according to the present invention all have analogous biological activity. Homologues, derivatives and fragments, in the present context, comprise at least 6 amino acids (as shown in SEQ ID NO: 2) corresponding to those of a naturally occurring antisecretory factor (AF) protein, which may be further modified by changing one or more amino acids in order to optimize the antisecretory factor's biological activity, without altering the essential function of the polypeptide and/or peptide.

By a derivative is in the present context intended a protein having equivalent activity and/or a functional equivalent activity to an antisecretory factor as defined herein, being derived from another substance either directly or by modification or partial substitution, wherein one or more amino acids have been substituted by another amino acid, which amino acid can be a modified or an unnatural amino acid. For example, the antisecretory factor derivatives according to the invention may comprise an N terminal and/or a C terminal protecting group. One example of an N terminal protecting group includes acetyl. One example of a C terminal protecting group includes amide.

Furthermore, any amino acid sequence being at least 70% identical, such as being at least 72%, 75%, 77%, 80%, 82%, 85%, 87%, 90%, 91%, 92%, 93%, 94%, 95%, 96%, 97%, 98%, or 99% identical with the amino acid sequence of an antisecretory factor (AF) protein, peptide, homologue, derivative and/or fragment according to the invention, is also considered to be inside the scope of the present invention.

By proteins, homologues, derivatives, peptides and/or fragment thereof having an amino acid sequence at least, for example 95% identical to a reference amino acid sequence, is intended that the amino acid sequence of e.g. the peptide is identical to the reference sequence, except that the amino acid sequence may include up to 5 point mutations per each 100 amino acids of the reference amino acid sequence. In other words, to obtain a polypeptide having an amino acid sequence at least 95% identical to a reference amino acid sequence, up to 5% of the amino acids in the reference sequence may be deleted or substituted with another amino acid, or a number of amino acids up to 5% of the total amino acids in the reference sequence may be inserted into the reference sequence. These mutations of the reference sequence may occur at the amino or carboxy terminal positions of the reference amino acid sequence or anywhere between those terminal positions, interspersed either individually among amino acids in the reference sequence or in one or more contiguous groups within the reference sequence.

In the present invention, a local algorithm program is best suited to determine identity. Local algorithm programs, (such as Smith Waterman) compare a subsequence in one sequence with a subsequence in a second sequence, and find the combination of sub-sequences and the alignment of those sub-sequences, which yields the highest overall similarity score. Internal gaps, if allowed, are penalized. Local algorithms work well for comparing two multi domain proteins, which have a single domain, or just a binding site in common.

Methods to determine identity and similarity are codified in publicly available programs. Preferred computer program methods to determine identity and similarity between two sequences include, but are not limited to, the GCG program package (Devereux, J et al (1994)) BLASTP, BLASTN, and FASTA (Altschul, S.F. et al (1990)). The BLASTX program is publicly available from NCBI and other sources (BLAST Manual, Altschul, S.F. et al, Altschul, S.F. et al (1990)). Each sequence analysis program has a default scoring matrix and default gap penalties. In general, a molecular biologist would be expected to use the default settings established by the software program used.

The antisecretory factor (AF) proteins or a peptide or a homologue, derivative and/or fragment thereof having equivalent activity as defined herein, can comprise 6 amino acids or more, such as 6-16 amino acids, such as 6, 7, 8, 9, 10, 11, 12, 13, 14, 15, 16, 17, 18, 19 or 20 amino acids or more. In other preferred embodiments the antisecretory factor consists of 42, 43, 45, 46, 51, 80, 128, 129 or 163 amino acids. In preferred embodiments the antisecretory factor (AF) protein, a homologue, derivative, peptide and/or fragment thereof, according to the present invention consists of 6, 7, 8 or 16 amino acids.

In another preferred embodiment, an antisecretory factor (AF) protein, a homologue, derivative, peptide and/or fragment thereof, according to the present invention consists of an amino acid sequence according to the following formulae:
X1-V-C-X2-X3-K-X4-R-X5, wherein X1 is I, amino acids 1-35 of SEQ ID NO 6, or is absent, X2 is H, R or K, X3 is S or L, X4 is T or A, X5 is amino acids 43-46, 43-51, 43-80 or 43-163 of SEQ ID NO 6, or is absent.

The antisecretory factor (AF) protein, a homologue, derivative, peptide and/or fragment thereof, according to the present invention, can be produced *in vivo* or *in vitro,* e.g. recombinantly, synthetically and/or chemically synthesized, and/or isolated from a naturally occurring source of antisecretory factors, such as from pig pituitary glands or bird's eggs. After production, the an antisecretory factor (AF) protein, a homologue, derivative, peptide and/or fragment thereof, according to the present invention may be further processed, such as by chemical or enzymatic cleavage to smaller antisecretory active fragments or by modification of amino acids. It is presently not possible to obtain antisecretory factor (AF)-protein in pure form by purification. It is however possible to produce a biologically active antisecretory factor protein recombinantly or synthetically as previously disclosed in WO 97/08202 and WO 05/030246. WO 97/08202 also discloses the production of biologically active fragments of this protein of 7-80 amino acids.

The antisecretory factor (AF) protein, a homologue, derivative, peptide and/or fragment thereof, according to the present invention may further comprise an N terminal and/or a C terminal protecting group. One example of an N terminal protecting group includes acetyl. One example of a C terminal protecting group includes amide.

In a preferred embodiment of the present invention the antisecretory factor (AF) protein, a homologue, derivative, peptide and/or fragment thereof, according to the present invention is selected among SEQ ID NO 1-6, i.e. VCHSKTRSNPENNVGL (SEQ ID NO 3, in this context also called AF-16), IVCHSKTR (SEQ ID NO 5), VCHSKTR (SEQ ID NO 4), CHSKTR (SEQ ID NO 2), HSKTR (SEQ ID NO 6), or the amino acid sequence of an antisecretory factor (AF) protein according to SEQ ID NO 1 using the common one letter abbreviations for amino acids. They have previously been disclosed in e.g. WO 05/030246. As specified in the accompanying sequence listing, some of the amino acids in the above-specified sequences may be replaced by other amino acids. In the following in this paragraph, the position of a particular amino acid in a particular amino acid sequence is calculated from the left, denoting the most N-terminal amino acid as being in position 1 in that particular sequence. Any amino acid substitution(s) as specified below may be performed independently of any other amino acid substitution(s) in that sequence. In SEQ ID NO 3, the C in position 2 may be replaced by S, H in position 3 may be replaced with R or K, S in position 4 may be replaced with L, and/or T in position 6 may be replaced with A. In SEQ ID NO 5, C in position 3 may be replaced by S, H in position 4 may be replaced by R or K, S in position 5 may be replaced by L, and/or T in position 7 may be replaced by A. In SEQ ID NO 4, C in position 2 may be replaced by S, H in position 3 may be replaced by R or K, S in position 4 may be replaced by L, and/or T in position 6 may be replaced by A. In SEQ ID NO 2, C in position 1 may be replaced by S, H in position 2 may be replaced by R or K, S in position 3 may be replaced by L, and/or T in position 5 may be replaced by A. In SEQ ID NO 6, H in position 1 may be replaced by R or K, S in position 2 may be replaced by L, and/or T in position 4 may be replaced by A.

Also intended by the present invention is the combination of two or more of any of the fragments according to SEQ ID NO 1-6.

Specific antisecretory factor (AF) proteins or peptides to be used according to and/or which are included by the present invention are selected from the group consisting of an antisecretory factor (AF) protein comprising an amino acid sequence as shown in SEQ ID NO: 1, an antisecretory factor (AF) protein which comprises an amino acid sequence as shown in SEQ ID NO: 2, an antisecretory factor (AF) protein which comprises an amino acid sequence as shown in SEQ ID NO: 3, an antisecretory factor (AF) protein which comprises an amino acid sequence as shown in SEQ ID NO: 4 and an antisecretory factor (AF) protein which comprises an amino acid sequence as shown in SEQ ID NO: 5. Furthermore, in yet another embodiment, the invention pertains to the use of an antisecretory factor (AF) protein which is a protein with an amino acid sequence as shown in SEQ ID NO:1, or a homologue, derivative and/or fragment thereof comprising amino acids 37-42 of SEQ ID NO:1.

In yet another embodiment, the invention relates to the use of a pharmaceutical composition as disclosed herein, which comprises two or more antisecretory factor (AF) proteins selected from the proteins as disclosed in SEQ ID NO: 1-6, and SEQ ID NO 1 or a homologue, derivative and/or fragment thereof comprising amino acids 37-42 of SEQ ID NO 1, or a sequence as disclosed by the general formulae described herein. Said sequences are all equally preferred to be used in the present invention.

WO 00/038535 discloses food products, enriched in antisecretory factor (AF) proteins as such, which are examples for suitable food, foodstuff and/or food supplements for use in the present context.

### Pharmaceutical composition

In one instance of the present invention, the pharmaceutical composition according to the invention further comprises a pharmaceutically acceptable excipient. The choice of pharmaceutically acceptable excipient and their optimum concentration for use according to the present invention can readily be determined by the skilled person by experimentation. Pharmaceutically acceptable excipients for use according to the present invention include solvents, buffering agents, preservatives, chelating agents, antioxidants, and stabilizers, emulsifying agents, suspending agents and/or diluents. The pharmaceutical compositions of the invention may be formulated according to conventional pharmaceutical practice, e.g. according to "Remington: The science and practice of pharmacy", 21st edition, ISBN 0-7817-4673-6 or "Encyclopedia of pharmaceutical technology", 2nd edition, ed. Swarbrick J., ISBN: 0-8247-2152-7. A pharmaceutically acceptable excipient is a substance that is substantially harmless to the individual to which the composition is to be administered. Such an excipient normally fulfills the requirements given by the national health authorities. Official pharmacopoeias such as e.g. the British Pharmacopoeia, the United States of America Pharmacopoeia and The European Pharmacopoeia set standards for pharmaceutically acceptable excipients.

The following is a review of relevant compositions for optional use in a pharmaceutical composition according to the invention. The review is based on the particular route of administration. However, it is appreciated that in those cases where a pharmaceutically acceptable excipient may be employed in different dosage forms or compositions, the application of a particular pharmaceutically acceptable excipient is not limited to a particular dosage form or of a particular function of the excipient. It should be emphasized that the invention is not limited to the use of the compositions mentioned in the following.

### Parenteral compositions:

For systemic application, the compositions according to the invention may contain conventional non-toxic pharmaceutically acceptable carriers and excipients, including micro spheres and liposomes.

The compositions for use according to the invention may include all kinds of solid, semisolid and fluid compositions.

The pharmaceutically acceptable excipients may include solvents, buffering agents, preservatives, chelating agents, antioxidants, and stabilizers, emulsifying agents, suspending agents and/or diluents. Examples of the different agents are given bellow.

### Example of various agents:

Examples of solvents include but are not limited to water, alcohols, blood, plasma, spinal fluid, ascites fluid and lymph fluid.

Examples of buffering agents include but are not limited to citric acid, acetic acid, tartaric acid, lactic acid, hydrogen phosphoric acid, bicarbonates, phosphates, diethylamide, etc.

Examples of chelating agents include but are not limited to EDTA and citric acid.

Examples of antioxidants include but are not limited to butylated hydroxyl anisole (BHA), ascorbic acid and derivatives thereof, tocopherol and derivatives thereof, cysteine, and mixtures thereof.

Examples of diluents and disintegrating agents include but are not limited to lactose, saccharose, emdex, calcium phosphates, calcium carbonate, calcium sulphate, mannitol, starches and microcrystalline cellulose.

Examples of binding agents include but are not limited to saccharose, sorbitol, gum acacia, sodium alginate, gelatine, chitosan, starches, cellulose, carboxymethylcellulose, methylcellulose, hydroxypropylcellulose, polyvinylpyrrolidone and polyetyleneglycol.

The pharmaceutical composition according to the invention is can in one context be administrated locally or via intravenous peripheral infusion or via intramuscular or subcutaneous injection into the patient or via buccal, pulmonary, nasal, cutaneous or oral routes. Furthermore, it is also possible to administer the pharmaceutical composition through a surgically inserted shunt into a cerebral ventricle of the patient.

In one embodiment, the pharmaceutical composition used according to the present invention is formulated for intraocular, local, intranasal, oral, subcutaneous and/or systemic administration. The chosen route of administration will vary depending on the condition of the patient to be treated and the patient's age and gender etc.
In a preferred embodiment, the composition of the invention is administrated by application as a suspension or, even more preferably, a powder for inhalation with a spray, aerosol, inhaler or nebulizer nasally and/or to the respiratory tract.

The administration of a powder comprising antisecretory factors has the additional advantages in terms of stability and dosage. A pharmaceutical composition according to the invention can also be topically applied, ocularly, nasally, orally, subcutaneously and/or systemically administered via blood vessels. In a preferred embodiment, the pharmaceutical composition is formulated for intravenous, intramuscular, local, oral or nasal administration. Typically, when used for topical application to the eye, the applied concentration in the composition of the invention is from 1 µg to 1 mg per application, preferably 50 - 500 µg, either as a single dose per day or repeated several times per day (multiple doses), but is not limited thereto.

Systemically administrated to the blood, the dose is within the range of 0.1 µg to 10 mg per application and kg body weight, such as 0.1 µg to 1 mg per application and kg body weight, preferably 1 - 500, such as 1 - 1000 µg/kg body weight. When egg yolk enriched in antisecretory factors is used according to the present invention, this formulation is preferably administered orally.

In one instance of the invention, said pharmaceutical composition further comprises a pharmaceutically acceptable excipient. Such an excipient may be any preferable excipient chosen to be appropriate for the specific purpose. Examples of excipients are disclosed herein.

### Method for treating glioblastoma

In one embodiment, the present invention relates to treating glioblastoma, characterized by administering an antisecretory factor (AF) protein as shown in SEQ ID NO: 1 (AF), and/or a homologue and/or fragment thereof having equivalent activity and comprising an amino acid sequence as shown in SEQ ID NO: 2 (AF-6), and/or a pharmaceutically active salt thereof to a patient in need thereof. Said treatment can in one embodiment of the present invention be used to facilitate an optimized drug uptake and delivery of a further pharmaceutical substance.

Said treating a mammalian suffering from glioblastoma can in a presently preferred embodiment comprise feeding a food, food stuff and/or food supplement to said patient and thereby inducing endogenous production of AF for facilitating an optimized drug uptake and delivery of a further pharmaceutical substance.

Said pharmaceutical substance and/or formulation is in the present context selected from the group consisting of anticancer drug, antitumor drug, radiation therapy, immunological substances and/or cells and antibiotic substance, a drug targeting posttraumatic injury, a drug targeting neurodegeneration, and a drug against inflammatory conditions. Said further pharmaceutical substance can be in the form of nano particles and/or formulations thereof in the treatment of glioblastoma (a GBM tumor).

Examples well known in the field are selected from but not limited to the group comprising an alkylating agent, such as temozolomide (Maity et al., 2008; Kaye and Laws, 2012) and Gliadel®, which is an implantable alkylating carmustine wafer. In addition, incorporated are antibodies to vascular endothelial growth factor.

In another, equally preferred embodiment, said method for treating a mammalian suffering from glioblastoma can comprise feeding egg yolk enriched in antisecretory factors to said patient and thereby optimizing drug uptake and delivery of a further pharmaceutical substance.

The present invention relates to treating glioblastoma (a GBM tumor) and/or for optimizing delivery and/or cellular uptake of a pharmaceutical substance and/or formulation, such as an anticancer drug, radiation therapy, therapy relying on immunological mechanisms or a gene delivery to a glioblastoma tumor cell, characterized by administering an antisecretory factor (AF) protein as shown in SEQ ID NO: 1 (AF), and/or a homologue and/or fragment thereof having equivalent activity and comprising an amino acid sequence as shown in SEQ ID NO: 2 (AF-6), and/or a pharmaceutically active salt thereof to a patient in need thereof.

The present application for the first time discloses a long sought for effective means and/or approach for treating GBM tumors. It surprisingly discloses symptomatic, curative and palliative therapies for glioblastoma, characterized by administering an antisecretory factor (AF) protein as shown in SEQ ID NO: 1 (AF), and/or a homologue and/or fragment thereof having equivalent activity and comprising an amino acid sequence as shown in SEQ ID NO: 2 (AF-6), and/or a pharmaceutically active salt thereof to a patient in need thereof. In particular, they can be used to optimize drug and gene delivery, as well as chemotherapy, immunotherapy and radiotherapy in the treatment of glioblastoma.

In particular, methods are disclosed for optimizing blood circulation and/or oxygen tension in a GBM tumor, facilitating effects of radiation therapy as well as immune therapy as well as methods for facilitating an optimized drug uptake and delivery of temozolomide optionally in combination with radiation to a GBM tumor cell.

Methods are disclosed for lowering a required drug dosage, alternatively for maximizing the dosage effect of said pharmaceutical substance and/or formulation characterized by administering an antisecretory factor (AF) protein as shown in SEQ ID NO: 1 (AF), and/or a homologue and/or fragment thereof having equivalent activity and comprising an amino acid sequence as shown in SEQ ID NO: 2 (AF-6), and/or a pharmaceutically active salt thereof to a patient in need thereof, which are used to minimize toxic or unwanted side effects of said pharmaceutical substance and/or formulation in the treatment of glioblastoma.

A food and/or food supplement, enriched in antisecretory factor (AF)-protein and/or homologue and/or fragment thereof, as described herein, is preferably provided as egg yolk enriched in naturally occurring antisecretory factors.

AF and the second or further pharmaceutical substance and/or formulation can be administered together or in alternating succession. They can be co-formulated or administered in separate formulations.

A method is envisioned for optimizing radiation therapy in the treatment of glioblastoma, characterized by administering an antisecretory factor (AF) protein as shown in SEQ ID NO: 1 (AF), and/or a homologue and/or fragment thereof having equivalent activity and comprising an amino acid sequence as shown in SEQ ID NO: 2 (AF-6), and/or a pharmaceutically active salt thereof to a patient in need thereof.

### Experimental section

### Example 1

### AF-16 (SEQ ID NO: 3)

The aim of this experiment was to investigate if administration of the peptide AF-16 lowered the high intracranial pressure prevailing in a brain with a glioblastoma. If so, the deleterious effects of the tumor induced brain edema and high intracranial pressure could be counteracted, a highly beneficial and desired effect.

Adult male Fisher 344 rats were purchased from Charles River, Germany. The body weight at the time for the experiments was 230-250 g. The animals had water and pelleted feed *ad libitum.* Permission to the experiments was granted by the *Regional Animal Experiments Ethical Committee,* and national and EU rules were followed.

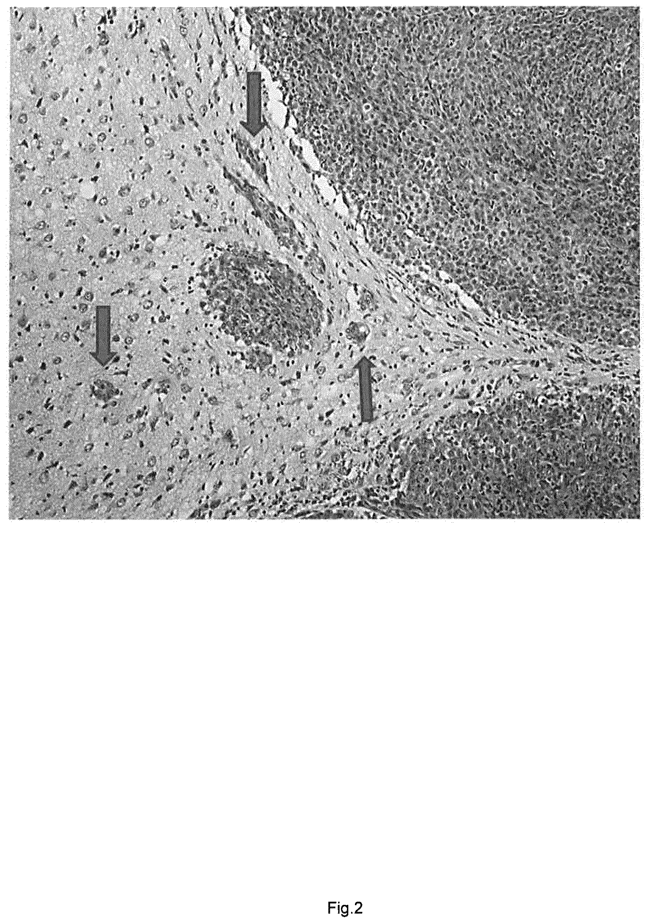

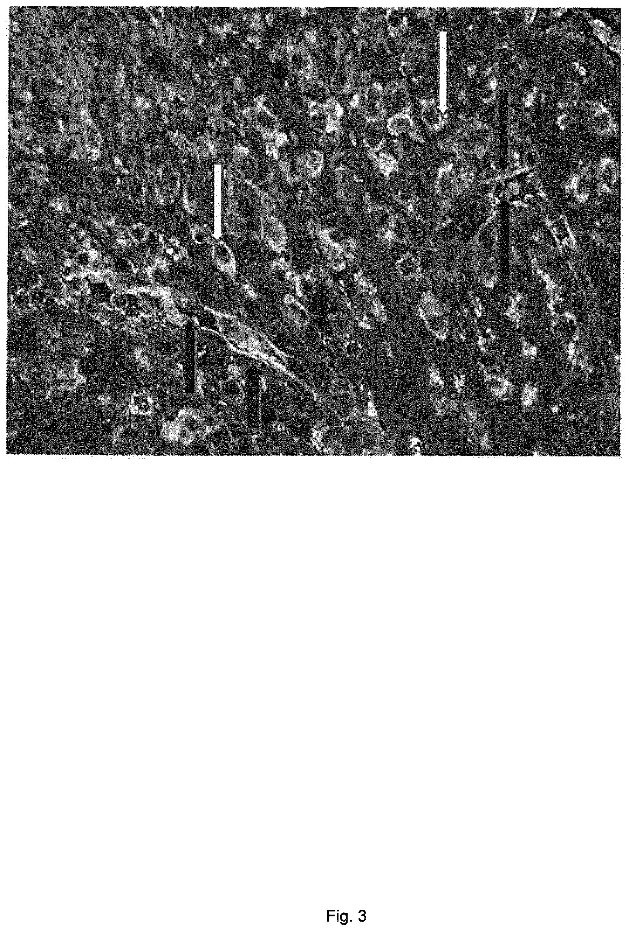

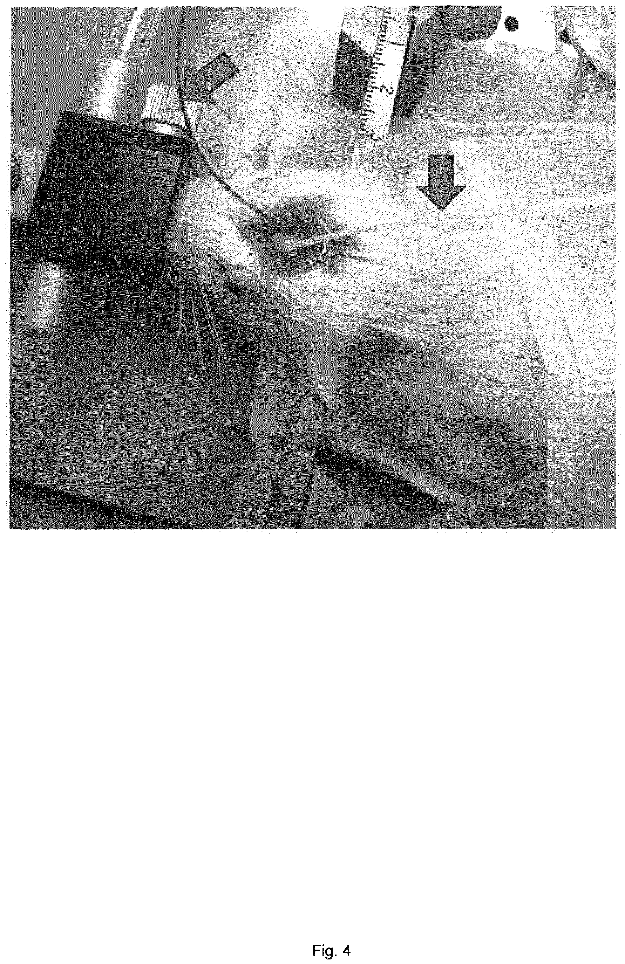

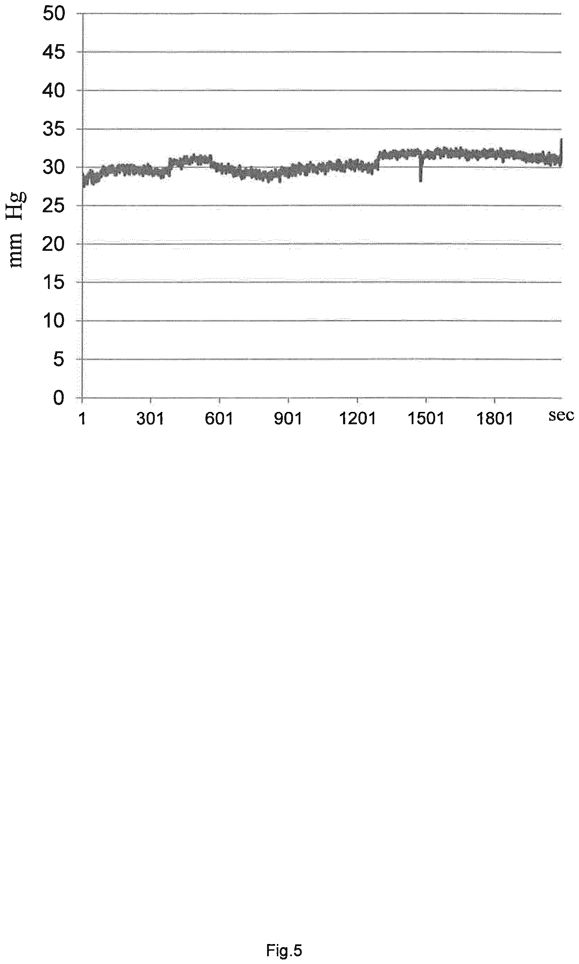

Cultured cells of the established glioma cell line RG2, clone N32, were stereotactically deposited in the right striatum of the brain of adult Fisher 344 rats (A.T. Aas, A Brun, C. Blennow, S. Strömblad and L.G. Salford (1995). The RG2 glioma model. J. Neuro-Oncology 23, 175-183; R. F. Barth and B. Kauer (299). Rat brain tumor models in experimental neuro-oncology: the C6, 9L, T9, RG2, F98, BT4C, RT-2 and CNS-1 gliomas. (J Neuro-Oncology 94, 299-312). After 18 - 22 days, a tumor, fulfilling established criteria for glioblastoma, was demonstrable in the right hemisphere, invading adjacent brain parenchyma (Figs. 1, 2). The cells in the extensively vascularized tumor were disclosed to be delimited by a glycocalyx, i.e. a thin layer of polysaccharides and proteoglycans, as revealed in Fig. 3. The expanding tumor enlarged the brain tissue, causing an increase of the intracranial pressure as the bony cranium did not allow any volume expansion. Fig. 4 is showing the implantation of pressure sensors (Samba 3200; Samba Sensors AB, V. Frölunda, Sweden), one in each hemisphere. The intracranial pressure was measured to be in the range of 25 - 50 mm Hg (Fig. 4), that means strikingly elevated as compared to normally 5.3 ± 2.1 mm Hg (H.-A. Hansson, M. Al-Olama, E. Jennische, K. Gatzinsky and S. Lange (2012). The peptide AF-16 and the AF protein counteract intracranial hypertension. Acta Neurochir. Suppl. 114, 377-382). Eventually, the intracranial pressure exceed 50 - 60 mm Hg, resulting in herniation of the brain, dislocations and impaired blood circulation, and death (Fig. 5).

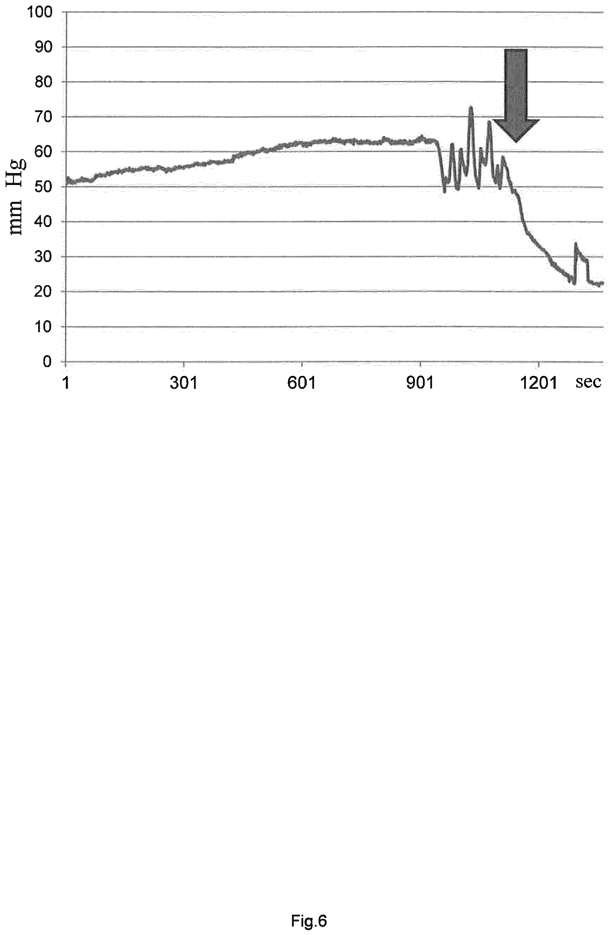

A single nasal instillation of 10 µL of a solution of the peptide AF-16 (1- 4 mg/kg body weight) in water resulted in 15 - 30 minutes in a reduction of the high intracranial pressure to an acceptable level, around 20 mm Hg or lower (Fig. 6). The intracranial pressure was raised both in the tumor affected right side and in the contralateral left side, and the pressure dropped to the same extent in either brain hemisphere. Concomitantly, any demonstrable neurological impairment resolved.

We conclude that the peptide AF-16 within 15 - 30 minutes counteracted the otherwise demonstrable elevation of the intracranial pressure in adult rats with an experimentally induced glioblastoma tumor. The beneficial effects lasted at least 4-6 h.

### Example 2

### AF- 6 (SEQ ID NO: 2)

The aim of this experiment was to investigate if administration of the peptide AF-6 lowered the high intracranial pressure prevailing in a brain with a glioblastoma. If so, the deleterious effects of the tumor induced brain edema and high intracranial pressure could be counteracted, a highly beneficial and desired effect.

Adult male Fisher 344 rats were purchased from Charles River, Germany. The body weight at the time for the experiments was 230-250 g. The animals had water and pelleted feed *ad libitum.* Permission to the experiments was granted by the *Regional Animal Experiments Ethical Committee,* and national and EU rules were followed.

Cultured cells of the established glioma cell line RG2, clone N32, were stereotactically deposited in the right striatum of the brain of adult Fisher 344 rats as described in *Example 1.* After 18 - 22 days a tumor, fulfilling established criteria for glioblastoma, was demonstrable in the right hemisphere, invading adjacent brain parenchyma.

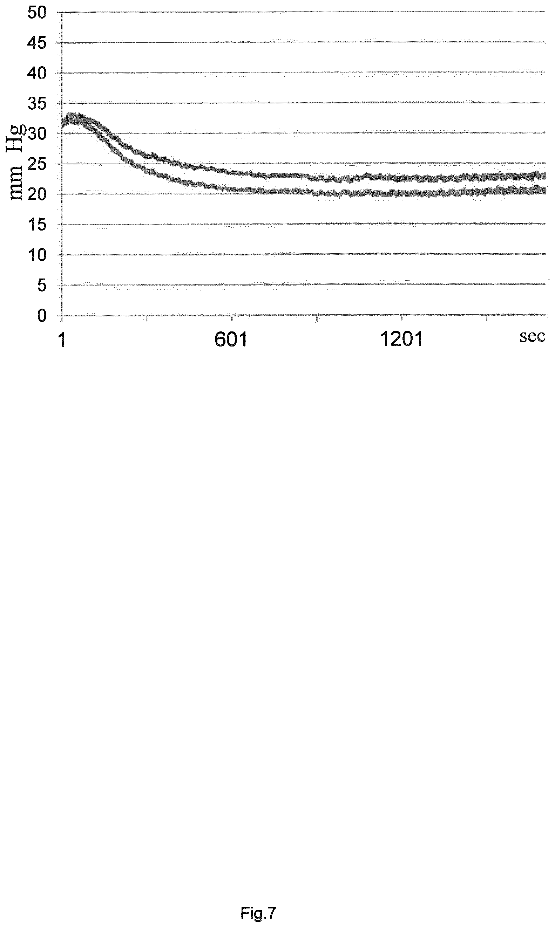

A single dose of the peptide AF-6 (1-2 mg/kg body weight, dissolved in water) was instilled in the nose of a Fisher rat with an experimental glioblastoma tumor of the RG2-N32 origin at day 21 after implantation. About 15 min later the intracranial pressure started to dropped from 25 - 27 mm Hg to 17.7 mm Hg (Fig. 7).

We conclude that the peptide AF-6 lowered the elevated intracranial pressure caused by an implanted glioblastoma tumor to an acceptable level, about 20 mm Hg or lower. No side effects could be disclosed.

### Example 3

### AF-6 (SEQ ID NO: 2)

The aim of this experiment was to investigate if administration of the peptide AF-16 lowered the high intracranial pressure prevailing in a mouse brain with a glioblastoma. If so, the deleterious effects of the tumor induced brain edema and high intracranial pressure could be counteracted, a highly beneficial and desired effect.

Adult C57/BI/6 mice were purchased from B & K, Sollentuna, Sweden. The body weight was 23 g and the animals had water and pelleted feed *ad libitum.* Permission to the experiments was granted by the *Regional Animal Experiments Ethical Committee,* and national and EU rules were followed.

The GL261 mouse glioma cell line was of C57BI/6 origin and cultured in the Rausing laboratory, BMC, Lund University, according to an established protocol (K.Enell Smith, S. Fritzell, W. Badn, S. Eberstål, S. Janelidze, E. Visse, A. Darabi and P. Siesjö (2008). Cure of established GL261 mouse gliomas after combined immunotherapy with GM-CSF and IFNγ is mediated by both CD8+ and CD4+ T-cells. Int. J. Cancer 124, 630-637).

C57/BI/6 mice had each 5.000 GL261 glioma cells in 5 µL tissue culture medium stereo tactically deposited in deep brain structure in the right brain hemisphere. An expanding tumor was formed and had 22 days later a diameter of 3-5 mm. The animal showed no obvious signs of sickness or motor disturbance when selected for experimental use.

The six AA peptide AF-6 (SEQ ID NO:2) (manufactured by solid phase synthesis by KJ Ross-Petersen ApS, Copenhagen, with >95 % purity and TFA as counter ion) was selected to be investigated for its ability to reduce the increased intracranial pressure (ICP) inevitably evolving at this stage of tumor development.

A mouse with a GL261 tumor in the right brain hemisphere was anaesthetized with isofluran and positioned in a stereotactic holder. The skin on the calvarium was incised, and soft tissue removed. Two holes, each with a diameter of 1 mm, were drilled in the right and the left parietal bones. A miniature fiber optic pressure transducer was inserted through each hole in the skull bone in the brain tissue, connected to a Samba 3200 pressure measuring equipment (Samba Samba Sensors AB, V. Frölunda, Sweden) and a computer. The sensors were calibrated prior to and after each measuring session.

The anaesthetized mouse had 5 µL of a 6 amino acid peptide, AF-6, dissolved in distilled water, instilled in each nostril while the animal was having its back downward. The dose was 5 mg per kg body weight. The continuous recording of the pressure in either hemisphere was thereafter started. The initial interstitial fluid pressure (IFP) in the right tumor hemisphere was 23 mm Hg and the ICP in the left hemisphere 19 mm Hg, which contrast to the ICP in a normal control mouse, about 6 mm Hg. Both the IFP and the ICP started to decrease after about 15 minutes. At 45 min after the nasal instillation of AF-6 the IFP in the right tumor hemisphere was 7 mm Hg while the IFP was 4 - 5 mm Hg in the left brain half. No side effects were noticed.

It is concluded that nasal instillation of the peptide AF-6 lowered the raised intracranial pressure evolving at a unilateral experimental brain tumor not only in the tumor-bearing hemisphere but also in the contralateral one. This means that AF -6 was effective in counteracting the raised complex pressure patterns evolving in brains with an expanding tumor.

### Example 4

### AF-16 (SEQ ID NO:3)

The aim of this experiment was to investigate if treatment with AF-16 increased the penetration of a marker through the tumor cells membrane and subsequent entrance and distribution in their nuclei. Doxorubicin was selected as a tracer for the reason that it is an established cytotoxic drug with a molecular mass of 543.52 g/mol, i.e. being considered as a fairly low molecular mass substance. Further, doxorubicin has a red color and when exposed to light emits a red fluorescence. Doxorubicin is strongly intercalating with the DNA in cell nuclei. This means that if a red fluorescence is observed in a cell nucleus the marker has been reached its target, the tumor cells. It is known that glioblastoma cells, in humans as in animals, prevent the entrance of chemotherapeutic substances into the tumor cells, and consequently no beneficial effects are attained. The most important barrier to the drug is prevailing at the surface of the tumor cells.

Adult male Fisher 344 rats were purchased from Charles River, Germany. The body weight at the time for the experiments was 230-250 g. The animals had water and pelleted feed *ad libitum.* Permission to the experiments was granted by the *Regional Animal Experiments Ethical Committee,* and national and EU rules were followed.

Cultured cells of the established glioma cell line RG2, clone N32, were stereotactically deposited in the right striatum of the brain of adult Fisher 344 rats as described in *Example* 1. After 20 days a tumor, fulfilling established criteria for being a glioblastoma, was present in the right brain hemisphere of either rat.

AF-16, 1 mg per kg body weight, dissolved in water and administrated as a single dose in a volume of 10 µL, was instilled in the nostrils of anaesthetized Fisher rats. One hour later, each animal had an intravenous injection of the tracer doxorubicin (Sigma) at a dose of 10 mg per kg body weight, and was eventually sacrificed 30 min later. Cryostat microtome sections were prepared of the brains, and examined with the aid of fluorescence microscope.

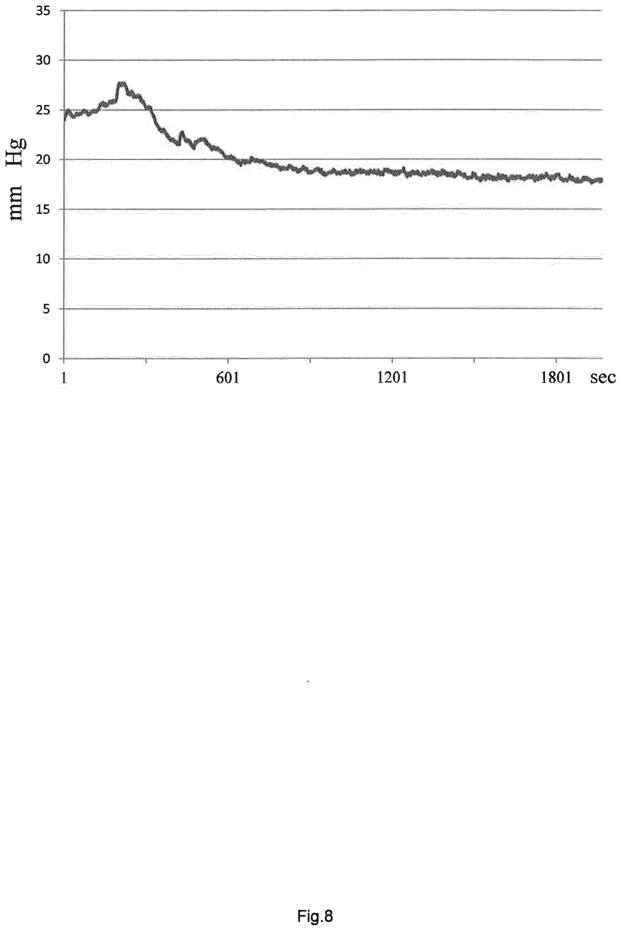

Fluorescence microscopy of section of rat brains with glioblastoma and treated by nasal administration of the vehicle, water, prior to the injection of the tracer doxorubicin showed only few scattered, red nuclei (Fig. 8). That means that very few doxorubicin molecules entered the tumor cells.

In contrast, pretreatment with AF-16 of rats with glioblastoma prior to the administration of doxorubicin resulted in a strong red staining of the tumor cell nuclei, and further that a large proportion of these cells were labeled (Fig. 9).

It is concluded that the administration of AF peptides strikingly improved the uptake of a marker for a cytotoxic drug, facilitated its distribution in the cells and the intercalation or binding of the compound to the DNA. Thus, AF peptides improved the access of chemotherapeutic agents to their target, the tumor cells.

### References

1. Lange, S., and Lönnroth, I. 2001. The antisecretory factor: synthesis, anatomical and cellular distribution, and biological action in experimental and clinical studies. Int. Rev. Cytol. 210, 39-75.
2. H.-A. Hansson, M. Al-Olama, E. Jennische, K. Gatzinsky and S. Lange (2012). The peptide AF-16 and the AF protein counteract intracranial hypertension. Acta Neurochir. Suppl. 114, 377-382).
3. K.Enell Smith, S. Fritzell, W. Badn, S. Eberstål, S. Janelidze, E. Visse, A. Darabi and P. Siesjö (2008). Cure of established GL261 mouse gliomas after combined immunotherapy with GM-CSF and IFNγ is mediated by both CD8+ and CD4+ T-cells. Int. J. Cancer 124, 630-637).
4. A.T. Aas, A Brun, C. Blennow, S. Strömblad and L.G. Salford (1995). The RG2 glioma model. J. Neuro-Oncology 23, 175-183;
5. R. F. Barth and B. Kauer (2009). Rat brain tumor models in experimental neuro-oncology: the C6, 9L, T9, RG2, F98, BT4C, RT-2 and CNS-1 gliomas. J Neuro-Oncology 94, 299-312.
6. Remington: The science and practice of pharmacy", 21st edition, ISBN 0-7817-4673-6 or "Encyclopedia of pharmaceutical technology", 2nd edition, ed. Swarbrick J., ISBN: 0-8247-2152-7.
7. WO 97/08202;
8. WO 05/030246
9. WO 97/08202
10. WO 97/08202
11. WO 98/21978
12. WO 00/038535.
13. WO 05/030246
14. WO 07/126364
15. WO 07/126363
16. WO 07/126365
17. WO 2010/093324

## Claims

1. An antisecretory factor (AF) protein as shown in SEQ ID NO: 1 (AF) and/or a homologue and/or fragment thereof having equivalent activity and comprising an amino acid sequence as shown in SEQ ID NO: 2 (AF-6), and/or a pharmaceutically active salt thereof, and/or a food and/or food supplement, enriched in said antisecretory factor (AF) protein and/or homologue and/or fragment thereof, for use in the treatment of glioblastoma.

2. An antisecretory factor (AF) protein and/or a homologue and/or fragment thereof and/or a pharmaceutically active salt thereof, and/or a food and/or food supplement, enriched in said antisecretory factor (AF) protein and/or homologue and/or fragment thereof for use according to claim 1, wherein the treatment is selected from the group consisting of curative therapy of glioblastoma, symptomatic therapy of glioblastoma, palliative therapy of glioblastoma, optimizing blood circulation and/or oxygen tension in a GBM tumor, and optimizing delivery and/or cellular uptake of a further pharmaceutical substance and/or formulation and/or gene delivery in the treatment of glioblastoma.

3. An antisecretory factor (AF) protein and/or a homologue and/or fragment thereof and/or a pharmaceutically active salt thereof, and/or a food and/or food supplement, enriched in said antisecretory factor (AF) protein and/or homologue and/or fragment thereof for use according to claim 2, wherein said further pharmaceutical substance and/or formulation is selected from the group consisting of an anticancer drug, a cytostaticum, genetic material, radiation therapy, antimicrobial substance, antibiotic substance, and an antiviral substance.

4. An antisecretory factor (AF) protein and/or a homologue and/or fragment thereof and/or a pharmaceutically active salt thereof, and/or a food and/or food supplement, enriched in said antisecretory factor (AF) protein and/or homologue and/or fragment thereof for use according to any of the preceding claims, which is formulated in a pharmaceutical composition comprising two or more antisecretory factor (AF) proteins and/or homologues and/or fragments thereof and/or pharmaceutically active salts thereof.

5. An antisecretory factor (AF) protein and/or a homologue and/or fragment thereof and/or a pharmaceutically active salt thereof, and/or a food and/or food supplement, enriched in said antisecretory factor (AF) protein and/or homologue and/or fragment thereof for use according to any of the preceding claims, wherein said pharmaceutical composition is formulated for intraocular, intranasal, oral, local, subcutaneous and/or systemic administration.

6. An antisecretory factor (AF) protein and/or a homologue and/or fragment thereof and/or a pharmaceutically active salt thereof, and/or a food and/or food supplement, enriched in said antisecretory factor (AF) protein and/or homologue and/or fragment thereof for use according to any of the preceding claims, wherein said pharmaceutical composition is formulated for administration as a spray, aerosol, inhaler and/or by a nebulizer.

7. An antisecretory factor (AF) protein and/or a homologue and/or fragment thereof and/or a pharmaceutically active salt thereof, and/or a food and/or food supplement, enriched in said antisecretory factor (AF) protein and/or homologue and/or fragment thereof for use according to any of the preceding claims, wherein the pharmaceutical composition and/or medical food is formulated for administration systemically to the blood at a dose of 0.1 µg to 10 mg per application and kg body weight and day, preferably 1-1000 µg per application and kg body weight and day and, wherein said administration is performed either as a single dose or as multiple daily applications.

8. An antisecretory factor (AF) protein and/or a homologue and/or fragment thereof and/or a pharmaceutically active salt thereof, and/or a food and/or food supplement, enriched in said antisecretory factor (AF) protein and/or homologue and/or fragment thereof for use according to any of the preceding claims, wherein the food and/or food supplement, enriched in the antisecretory factor (AF) protein and/or homologue and/or fragment thereof according to any of the preceding claims, is provided as egg yolk enriched in antisecretory factors.

9. A pharmaceutical composition comprising an antisecretory factor (AF) protein as shown in SEQ ID NO: 1 (AF), and/or a homologue and/or fragment thereof having equivalent activity and comprising an amino acid sequence as shown in SEQ ID NO: 2 (AF-6), and/or a pharmaceutically active salt thereof in combination with temozolomide.

10. A pharmaceutical composition according to claim 10, wherein the fragment of an antisecretory factor (AF) protein is AF-16 (SEQ ID NO:3) or AF-6 (SEQ ID NO:2).

11. A pharmaceutical composition comprising a food and/or food supplement, enriched in an antisecretory factor (AF) protein as shown in SEQ ID NO: 1 (AF), and/or a homologue and/or fragment thereof having equivalent activity and comprising an amino acid sequence as shown in SEQ ID NO: 2 (AF-6), and/or a pharmaceutically active salt thereof, in combination with temozolomide.

12. A pharmaceutical composition according to claim 12, wherein the food and/or food supplement is egg yolk enriched in naturally occurring antisecretory factors (NASPs).

13. A pharmaceutical composition according to any of claims 10- 13 for use in medicine.

14. A pharmaceutical composition according to any of claims 10- 13 for use in treating glioblastoma.

## Patentansprüche

1. Protein des antisekretorischen Faktors (AF), wie in SEQ ID NR.: 1 (AF) gezeigt, und/oder ein Homologes und/oder Fragment davon mit äquivalenter Aktivität und umfassend eine Aminosäuresequenz, wie in SEQ ID NR.: 2 (AF-6) gezeigt, und/oder ein pharmazeutisch wirksames Salz davon und/oder ein Lebensmittel und/oder eine Nahrungsergänzung, angereichert mit dem Protein des antisekretorischen Faktors (AF) und/oder Homologen und/oder Fragment davon, zur Verwendung bei der Behandlung von Glioblastom.

2. Protein des antisekretorischen Faktors (AF) und/oder ein Homologes und/oder Fragment davon und/oder ein pharmazeutisch wirksames Salz davon und/oder ein Lebensmittel und/oder eine Nahrungsergänzung, angereichert mit dem Protein des antisekretorischen Faktors (AF) und/oder Homologen und/oder Fragment davon, zur Verwendung nach Anspruch 1, wobei die Behandlung ausgewählt ist aus der Gruppe, bestehend aus heilender Therapie von Glioblastom, symptomatischer Therapie von Glioblastom, palliativer Therapie von Glioblastom, Optimieren des Blutkreislaufs und/oder der Sauerstoffspannung in einem GBM-Tumor und Optimieren der Abgabe und/oder zellulären Aufnahme einer weiteren pharmazeutischen Substanz und/oder Formulierung und/oder Genabgabe bei der Behandlung von Glioblastom.

3. Protein des antisekretorischen Faktors (AF) und/oder ein Homologes und/oder Fragment davon und/oder ein pharmazeutisch wirksames Salz davon und/oder ein Lebensmittel und/oder eine Nahrungsergänzung, angereichert mit dem Protein des antisekretorischen Faktors (AF) und/oder Homologen und/oder Fragment davon, zur Verwendung nach Anspruch 2, wobei die weitere pharmazeutische Substanz und/oder Formulierung ausgewählt ist aus der Gruppe, bestehend aus einem Antikrebsarzneistoff, einem Zytostatikum, genetischem Material, Strahlungstherapie, antimikrobieller Substanz, antibiotischer Substanz und einer antiviralen Substanz.

4. Protein des antisekretorischen Faktors (AF) und/oder ein Homologes und/oder Fragment davon und/oder ein pharmazeutisch wirksames Salz davon und/oder ein Lebensmittel und/oder eine Nahrungsergänzung, angereichert mit dem Protein des antisekretorischen Faktors (AF) und/oder Homologen und/oder Fragment davon, zur Verwendung nach einem der vorstehenden Ansprüche, das in einer pharmazeutischen Zusammensetzung formuliert wird, umfassend zwei oder mehrere Proteine des antisekretorischen Faktors (AF) und/oder Homologe und/oder Fragmente davon und/oder pharmazeutisch wirksame Salze davon.

5. Protein des antisekretorischen Faktors (AF) und/oder ein Homologes und/oder Fragment davon und/oder ein pharmazeutisch wirksames Salz davon und/oder ein Lebensmittel und/oder eine Nahrungsergänzung, angereichert mit dem Protein des antisekretorischen Faktors (AF) und/oder Homologen und/oder Fragment davon, zur Verwendung nach einem der vorstehenden Ansprüche, wobei die pharmazeutische Zusammensetzung zur intraokularen, intranasalen, oralen, örtlichen, subkutanen und/oder systemischen Verabreichung formuliert wird.

6. Protein des antisekretorischen Faktors (AF) und/oder ein Homologes und/oder Fragment davon und/oder ein pharmazeutisch wirksames Salz davon und/oder ein Lebensmittel und/oder eine Nahrungsergänzung, angereichert mit dem Protein des antisekretorischen Faktors (AF) und/oder Homologen und/oder Fragment davon, zur Verwendung nach einem der vorstehenden Ansprüche, wobei die pharmazeutische Zusammensetzung zur Verabreichung als ein Spray, Aerosol, Inhalator und/oder durch einen Zerstäuber formuliert wird.

7. Protein des antisekretorischen Faktors (AF) und/oder ein Homologes und/oder Fragment davon und/oder ein pharmazeutisch wirksames Salz davon und/oder ein Lebensmittel und/oder eine Nahrungsergänzung, angereichert mit dem Protein des antisekretorischen Faktors (AF) und/oder Homologen und/oder Fragment davon zur Verwendung nach einem der vorstehenden Ansprüche, wobei die pharmazeutische Zusammensetzung und/oder das medizinische Lebensmittel zur Verabreichung systemisch an das Blut bei einer Dosis von 0,1 µg bis 10 mg pro Applikation und kg Körpergewicht und Tag, vorzugsweise 1-1000 µg pro Applikation und kg Körpergewicht und Tag, formuliert wird und wobei die Verabreichung entweder als eine Einzeldosis oder als mehrfache tägliche Applikationen durchgeführt wird.

8. Protein des antisekretorischen Faktors (AF) und/oder ein Homologes und/oder Fragment davon und/oder ein pharmazeutisch wirksames Salz davon und/oder ein Lebensmittel und/oder eine Nahrungsergänzung, angereichert mit dem Protein des antisekretorischen Faktors (AF) und/oder Homologen und/oder Fragment davon, zur Verwendung nach einem der vorstehenden Ansprüche, wobei das Lebensmittel und/oder die Nahrungsergänzung, angereichert mit dem Protein des antisekretorischen Faktors (AF) und/oder Homologen und/oder Fragment davon nach einem der vorstehenden Ansprüche, als Eidotter, angereichert in antisekretorischen Faktoren, bereitgestellt wird.

9. Pharmazeutische Zusammensetzung, umfassend ein Protein des antisekretorischen Faktors (AF), wie in SEQ ID NR.: 1 (AF) gezeigt, und/oder ein Homologes und/oder Fragment davon mit äquivalenter Aktivität und umfassend eine Aminosäuresequenz, wie in SEQ ID NR.: 2 (AF-6) gezeigt, und/oder ein pharmazeutisch wirksames Salz davon in Kombination mit Temozolomid.

10. Pharmazeutische Zusammensetzung nach Anspruch 9, wobei das Fragment von einem Protein des antisekretorischen Faktors (AF) AF-16 (SEQ ID NR.:3) oder AF-6 (SEQ ID NR.:2) ist.

11. Pharmazeutische Zusammensetzung, umfassend ein Lebensmittel und/oder eine Nahrungsergänzung, angereichert mit einem Protein des antisekretorischen Faktors (AF), wie in SEQ ID NR.: 1 (AF) gezeigt, und/oder ein Homologes und/oder Fragment davon mit äquivalenter Aktivität und umfassend eine Aminosäuresequenz, wie in SEQ ID NR.: 2 (AF-6) gezeigt, und/oder ein pharmazeutisch wirksames Salz davon, in Kombination mit Temozolomid.

12. Pharmazeutische Zusammensetzung nach Anspruch 11, wobei das Lebensmittel und/oder die Nahrungsergänzung Eidotter, angereichert mit natürlich vorkommenden antisekretorischen Faktoren (NASPs), ist.

13. Pharmazeutische Zusammensetzung nach einem der Ansprüche 9-12 zur Verwendung in der Medizin.

14. Pharmazeutische Zusammensetzung nach einem der Ansprüche 9-12 zur Verwendung bei der Behandlung von Glioblastom.

## Revendications

1. Protéine du facteur antisécrétoire (AF) comme indiqué dans SEQ ID NO: 1 (AF) et/ou un homologue et/ou un fragment de celle-ci ayant une activité équivalente et comprenant une séquence d'acides aminés comme indiqué dans SEQ ID NO: 2 (AF-6), et/ou un sel pharmaceutiquement actif de celle-ci, et/ou un aliment et/ou un complément alimentaire, enrichi en ladite protéine du facteur antisécrétoire (AF) et/ou un homologue et/ou un fragment de celle-ci, pour une utilisation dans le traitement du glioblastome.

2. Protéine de facteur antisécrétoire (AF) et/ou un homologue et/ou un fragment de celle-ci et/ou un sel pharmaceutiquement actif de celle-ci et/ou un aliment et/ou un complément alimentaire, enrichi en ladite protéine du facteur antisécrétoire (AF) et/ou un homologue et/ou un fragment de celle-ci, pour une utilisation selon la revendication 1, dans laquelle le traitement est choisi dans le groupe consistant en un traitement curatif du glioblastome, un traitement symptomatique du glioblastome, un traitement palliatif du glioblastome, une optimisation de la circulation sanguine et/ou la tension de l'oxygène dans une tumeur GBM, une optimisation de l'administration et/ou de l'absorption cellulaire d'une autre substance pharmaceutique et/ou une formulation et/ou une administration d'un gène dans le traitement du glioblastome.

3. Protéine de facteur antisécrétoire (AF) et/ou un homologue et/ou un fragment de celle-ci et/ou un sel pharmaceutiquement actif de celle-ci et/ou un aliment et/ou un complément alimentaire, enrichi en ladite protéine du facteur antisécrétoire (AF) et/ou un homologue et/ou un fragment de celle-ci, pour une utilisation selon la revendication 2, dans laquelle ladite autre substance pharmaceutique et/ou formulation supplémentaire est choisie dans le groupe consistant en un médicament anticancéreux, un cytostatique, un matériel génétique, une radiothérapie, une substance antimicrobienne, une substance antibiotique, une substance antivirale.

4. Protéine de facteur antisécrétoire (AF) et/ou un homologue et/ou un fragment de celle-ci et/ou un sel pharmaceutiquement actif de celle-ci et/ou un aliment et/ou un complément alimentaire, enrichi en ladite protéine du facteur antisécrétoire (AF) et/ou un homologue et/ou un fragment de celle-ci, pour une utilisation selon l'une quelconque des revendications précédentes, qui est formulée dans une composition pharmaceutique comprenant deux ou plusieurs protéines de facteur antisécrétoire (AF) et/ou des homologues et/ou des fragments de celles-ci et/ou leurs sels pharmaceutiquement actifs.

5. Protéine de facteur antisécrétoire (AF) et/ou un homologue et/ou un fragment de celle-ci et/ou un sel pharmaceutiquement actif de celle-ci et/ou un aliment et/ou un complément alimentaire, enrichi en ladite protéine du facteur antisécrétoire (AF) et/ou un homologue et/ou un fragment de celle-ci, pour une utilisation selon l'une quelconque des revendications précédentes, dans laquelle ladite composition pharmaceutique est formulée pour une administration intraoculaire, intranasale, orale, locale, sous-cutanée et/ou systémique.

6. Protéine de facteur antisécrétoire (AF) et/ou un homologue et/ou un fragment de celle-ci et/ou un sel pharmaceutiquement actif de celle-ci et/ou un aliment et/ou un complément alimentaire, enrichi en ladite protéine du facteur antisécrétoire (AF) et/ou un homologue et/ou un fragment de celle-ci, pour une utilisation selon l'une quelconque des revendications précédentes, dans laquelle ladite composition pharmaceutique est formulée pour l'administration en tant que pulvérisation, aérosol, inhalateur et/ou par un nébuliseur.

7. Protéine de facteur antisécrétoire (AF) et/ou un homologue et/ou un fragment de celle-ci et/ou un sel pharmaceutiquement actif de celle-ci et/ou un aliment et/ou un complément alimentaire, enrichi en ladite protéine du facteur antisécrétoire (AF) et/ou un homologue et/ou un fragment de celle-ci, pour une utilisation selon l'une quelconque des revendications précédentes, dans laquelle ladite composition pharmaceutique et/ou l'alicament est (sont) formulé(e)(s) pour une administration systématiquement dans le sang à une dose de 0,1 µg à 10 mg par application et kg de poids corporel et jour, de préférence 1 à 1000 µg par application et kg de poids corporel et de jour, et dans laquelle ladite administration est effectuée soit en une dose unique, soit en tant qu'applications quotidiennes multiples.

8. Protéine de facteur antisécrétoire (AF) et/ou un homologue et/ou un fragment de celle-ci et/ou un sel pharmaceutiquement actif de celle-ci et/ou un aliment et/ou un complément alimentaire, enrichi en ladite protéine du facteur antisécrétoire (AF) et/ou un homologue et/ou un fragment de celle-ci, pour une utilisation selon l'une quelconque des revendications précédentes, dans laquelle l'aliment et/ou le complément alimentaire, enrichi en ladite protéine du facteur antisécrétoire (AF) et/ou un homologue et/ou un fragment de celle-ci, est fourni comme le jaune d'oeuf enrichi en facteurs antisécrétoires.

9. Composition pharmaceutique comprenant une protéine de facteur antisécrétoire (AF) comme indiqué dans SEQ ID NO: 1 (AF), et/ou un homologue et/ou un fragment de celle-ci ayant une activité équivalente et comprenant une séquence d'acides aminés comme indiqué dans SEQ ID NO: 2 (AF-6), et/ou un sel pharmaceutiquement actif de celle-ci en combinaison avec le temozolomide.

10. Composition pharmaceutique selon la revendication 9, dans laquelle le fragment d'une protéine du facteur antisécrétoire (AF) est AF-16 (SEQ ID NO: 3) ou AF-6 (SEQ ID NO: 2).

11. Composition pharmaceutique comprenant un aliment et/ou un complément alimentaire enrichi en une protéine de facteur antisécrétoire (AF) comme indiqué dans SEQ ID NO: 1 (AF) et/ou un homologue et/ou un fragment de celle-ci ayant une activité équivalente et comprenant une séquence d'acides aminés comme indiqué dans SEQ ID NO: 2 (AF-6), et/ou un sel pharmaceutiquement actif de celle-ci, en combinaison avec le temozolomide.

12. Composition pharmaceutique selon la revendication 11, dans laquelle l'aliment et/ou le complément alimentaire est le jaune d'oeuf enrichi en facteurs antisécrétoires naturels (NASP).

13. Composition pharmaceutique selon l'une quelconque des revendications 9-12, pour une utilisation en médecine.

14. Composition pharmaceutique selon l'une quelconque des revendications 9-12, pour une utilisation dans le traitement du glioblastome.


### SEQUENCE LISTING

<110> Lantmännen AS Faktor AB
<120> Use of antisecretory factor (AF) in glioblastoma treatment
<130> PS54487PC00
<160> 6
<170> FastSEQ for Windows Version 4.0
<210> 1
   <211> 382
   <212> PRT
   <213> human
<400> 1
   Met Val Leu Glu Ser Thr Met Val Cys Val Asp Asn Ser Glu Tyr Met
    1               5                  10                  15
   Arg Asn Gly Asp Phe Leu Pro Thr Arg Leu Gln Ala Gln Gln Asp Ala
               20                  25                  30
   Val Asn Ile Val Cys His Ser Lys Thr Arg Ser Asn Pro Glu Asn Asn
           35                  40                  45
   Val Gly Leu Ile Thr Leu Ala Asn Asp Cys Glu Val Leu Thr Thr Leu
       50                  55                  60
   Thr Pro Asp Thr Gly Arg Ile Leu Ser Lys Leu His Thr Val Gln Pro
   65                  70                  75                  80
   Lys Gly Lys Ile Thr Phe Cys Thr Gly Ile Arg Val Ala His Leu Ala
                   85                  90                  95
   Leu Lys His Arg Gln Gly Lys Asn His Lys Met Arg Ile Ile Ala Phe
               100                 105                 110
   Val Gly Ser Pro Val Glu Asp Asn Glu Lys Asp Leu Val Lys Leu Ala
           115                 120                 125
   Lys Arg Leu Lys Lys Glu Lys Val Asn Val Asp Ile Ile Asn Phe Gly
       130                 135                 140
   Glu Glu Glu Val Asn Thr Glu Lys Leu Thr Ala Phe Val Asn Thr Leu
   145                 150                 155                 160
   Asn Gly Lys Asp Gly Thr Gly Ser His Leu Val Thr Val Pro Pro Gly
                   165                 170                 175
   Pro Ser Leu Ala Asp Ala Leu Ile Ser Ser Pro Ile Leu Ala Gly Glu
               180                 185                 190
   Gly Gly Ala Met Leu Gly Leu Gly Ala Ser Asp Phe Glu Phe Gly Val
           195                 200                 205
   Asp Pro Ser Ala Asp Pro Glu Leu Ala Leu Ala Leu Arg Val Ser Met
       210                 215                 220
   Glu Glu Gln Arg His Ala Gly Gly Gly Ala Arg Arg Ala Ala Arg Ala
   225                 230                 235                 240
   Ser Ala Ala Glu Ala Gly Ile Ala Thr Thr Gly Thr Glu Asp Ser Asp
                   245                 250                 255
   Asp Ala Leu Leu Lys Met Thr Ile Ser Gln Gln Glu Phe Gly Arg Thr
               260                 265                 270
   Gly Leu Pro Asp Leu Ser Ser Met Thr Glu Glu Glu Gln Ile Ala Tyr
           275                 280                 285
   Ala Met Gln Met Ser Leu Gln Gly Ala Glu Phe Gly Gln Ala Glu Ser
       290                 295                 300
   Ala Asp Ile Asp Ala Ser Ser Ala Met Asp Thr Ser Glu Pro Ala Lys
   305                 310                 315                 320
   Glu Glu Asp Asp Tyr Asp Val Met Gln Asp Pro Glu Phe Leu Gln Ser
                   325                 330                 335
   Val Leu Glu Asn Leu Pro Gly Val Asp Pro Asn Asn Glu Ala Ile Arg
               340                 345                 350
   Asn Ala Met Gly Ser Leu Pro Pro Arg Pro Pro Arg Thr Ala Arg Arg
           355                 360                 365
   Thr Arg Arg Arg Lys Thr Arg Ser Glu Thr Gly Gly Lys Gly
       370                 375                 380
<210> 2
   <211> 6
   <212> PRT
   <213> Artificial sequence
<220>
   <221> VARIANT
   <222> 5
   <223> may be replaced by A
<221> VARIANT
   <222> 2
   <223> may be replaced by R or K
<221> VARIANT
   <222> 3
   <223> may be replaced by L
<221> VARIANT
   <222> 1
   <223> may be replaced by S
<400> 2
   Cys His Ser Lys Thr Arg
    1               5
<210> 3
   <211> 16
   <212> PRT
   <213> artificial sequence
<220>
   <221> VARIANT
   <222> 6
   <223> may be replaced with A
<221> VARIANT
   <222> 3
   <223> may be replaced with R or K
<221> VARIANT
   <222> 4
   <223> may be replaced with L
<221> VARIANT
   <222> 2
   <223> may be replaced by S
<400> 3
   Val Cys His Ser Lys Thr Arg Ser Asn Pro Glu Asn Asn Val Gly Leu
   1 5 10 15
<400> 4
   <211> 7
   <212> PRT
   <213> Artificial sequence
<220>
   <221> VARIANT
   <222> 6
   <223> may be replaced with A
<221> VARIANT
   <222> 3
   <223> may be replaced with R or K
<221> VARIANT
   <222> 4
   <223> may be replaced with L
<221> VARIANT
   <222> 2
   <223> may be replaced by S
<400> 4
<210> 5
   <211> 8
   <212> PRT
   <213> Artificial Sequence
<220>
   <221> VARIANT
   <222> 7
   <223> may be replaced with A
<221> VARIANT
   <222> 4
   <223> may be replaced with R or K
<221> VARIANT
   <222> 5
   <223> may be replaced with L
<221> VARIANT
   <222> 3
   <223> may be replaced by S
<400> 5
   Ile Val Cys His Ser Lys Thr Arg
   1 5
<210> 6
   <211> 5
   <212> PRT
   <213> Artificial Sequence
<220> Active Peptide
   <221> VARIANT
   <222> 1
   <223> may be replaced by R or K
<221> VARIANT
   <222> 2
   <223> may be replaced by L
<221> VARIANT
   <222> 4
   <223> may be replaced by A
<400> 6
   His Ser Lys Thr Arg
   1 5